# EUROPEAN PATENT SPECIFICATION

(11) **EP 3 570 030 B1**
(45) Date of publication and mention of the grant of the patent: **16.03.2022**
(21) Application number: 18172401.4
(22) Date of filing: 15.05.2018
(51) Int. Cl.: G01N 33/543, B01L 3/00, C12Q 1/68

(54) **ANALYZER DEVICE FOR IN VITRO DIAGNOSTICS**
ANALYSATORVORRICHTUNG FÜR IN-VITRO-DIAGNOSTIK
DISPOSITIF D'ANALYSE POUR DIAGNOSTICS IN VITRO

(43) Date of publication of application: 20.11.2019
(73) Proprietor: Siemens Healthcare GmbH, 91052 Erlangen (DE)
(72) Inventor: Kuth, Rainer, 91315 Höchstadt (DE); Würstle, Maximilian, 91083 Baiersdorf (DE)

(56) References cited:
- WO-A1-2015/139022
- WO-A1-2017/091718
- PARDEE KEITH ET AL: "Rapid, Low-Cost Detection of Zika Virus Using Programmable Biomolecular Components", CELL, CELL PRESS, AMSTERDAM, NL, [Online] vol. 165, no. 5, 6 May 2016 (2016-05-06), pages 1255-1266, XP029552292, ISSN: 0092-8674, DOI: 10.1016/J.CELL.2016.04.059 Retrieved from the Internet: URL:http://www.cell.com/cell/fulltext/S009 2-8674(16)30505-0> [retrieved on 2016-05-06]
- KAUSHIK AJEET ET AL: "Electrochemical Biosensors for Early Stage Zika Diagnostics", TRENDS IN BIOTECHNOLOGY, vol. 35, no. 4, April 2017 (2017-04), pages 308-317, XP029948711, ISSN: 0167-7799, DOI: 10.1016/J.TIBTECH.2016.10.001

## Description

### TECHNICAL FIELD

The present invention relates to an analyzer device for analyzing a sample comprising (i) at least two nested recursive structure elements; (ii) wherein each of the recursive structure elements has at least one connection to at least one of the other recursive structure elements; (iii) at least one measurement functionality; wherein said analyzer device is configured to allow, via said connections, a displacement of the sample and of material present in a recursive structure element, such as liquid material, to the next recursive structure elements by gravity in the form of a rotation of the analyzer device in a 3D-coordinate system or, optionally, with additional forces such as capillary motion or air or liquid pressure, wherein at least one of the recursive structure elements comprises at least one opening at the surface, configured to receive and/or release the sample for analyzing, wherein the recursive structure elements are suitable to hold liquids within, wherein all recursive structure elements are part of a fluid communication system providing direct or indirect connections between said recursive structure elements, wherein the analyzer device comprises within at least one recursive structure element or in separate recursive structure elements a measuring zone implementing the measurement functionality; and wherein said connections have the form of straight or curved elongations with two openings. Also provided is a method for producing the analyzer device via 3D printing, a system comprising the device and a reagent pack, a method for in vitro diagnosis of viral or bacterial infections or cancer using the device, as well as the use of the analyzing device for the diagnostic analysis of a sample, in particular on the basis of the CRISPR/Cas system.

### BACKGROUND

Typical approaches in laboratory medicine are based on complex diagnostics systems performed on sophisticated and expensive analytical devices. Medical centers, as well as self-employed medical practitioners are often dependent on specialized external laboratory experts providing diagnostic analyses for several clinically relevant parameters in a high-throughput manner. The workflow used normally includes the shipment of patient samples to the laboratory involving a complex logistic infrastructure. Moreover, in order to obtain reliable results in medical measurements, the samples usually have to be specifically treated, preserved or cooled requiring a cold chain or at least predefined transportation conditions.

Moreover, in vitro-diagnostic manufacturers recently shifted to the delivery of point-of-care scanners. These scanners are compact and designed for operation at a decentralized and local medical office. However, the scanners are typically dedicated to only few test assays. Thus, if a new type of disease and a corresponding test emerges the point-of care scanners are frequently not suited to process such a new assay or to perform the test.

Medical practitioners who work in locations remote from such highly specialized laboratories require suitable alternatives in order to analyze patient samples in an inexpensive, simple, readily available and decentralized manner.

Already known in the art are inexpensive and simple in vitro diagnostic test strips such as test strips for blood glucose self-monitoring using blood as sample or test strips for urine analysis e.g. for the rapid determination of ascorbic acid, bilirubin, blood, glucose, ketone, leukocytes, nitrite, pH, protein, and urobilinogen from urine. Test strips are usually easy to handle and are applicable, even for a person without prior medical knowledge. However, no complex diagnostic assays can be performed on test strips and the assays cannot be adapted flexibly to any new diseases that may arise.

Document WO 2017/091718 A1 discloses a multicompartment layered and stackable microfluidic bioreactors.

Document WO 2015/139022 discloses a microfluidic system and method for real-time measurement of antibody-antigen binding and analyte detection.

Kaushik et al., Trends in Biotechnology, 35, 4, 308-317 discloses electrochemical biosensors for early stage Zika diagnostics.

The recent Zika virus outbreak highlights the need for low-cost diagnostics that can be rapidly developed for distribution and use in pandemic regions. The rapid design, assembly, and validation of cell-free sensors for the detection of the Zika virus RNA genome has been made possible using a CRISPR/Cas method (Clustered Regularly Interspaced Short Pal-indromic Repeats). For example, Pardee et al. 2016, Cell, 165, 1255-1266 discloses that clinically relevant concentrations of Zika virus sequences can be detected by linking isothermal RNA amplification to toehold switch RNA sensors, i.e. programmable synthetic riboregulators that control the translation of a gene via the binding of a transacting trigger RNA. To identify the viral strain in a single-base resolution, the detection method is combined with a CRISPR/Cas9-based module. This NASBA-CRISPR Cleavage (NASBACC) approach allows to discriminate between viral strains with single-base resolution.

Recently, a further CRISPR/Cas-based diagnostic system called SHERLOCK (Specific High Sensitivity Enzymatic Reporter UnLOCKing) has been developed (Gootenberg et al., 2017, Science, 356(6336), 438-442; Gootenberg et al., 2018, Science, 10.1126/science. aaq0179). SHERLOCK combines isothermal pre-amplification with Cas13 to detect single molecules of RNA or DNA and is capable of discriminating between RNAs or DNAs that differ by a single nucleotide at low concentration. Thus, the SHERLOCK approach allows to detect, inter alia, Dengue or Zika virus ssRNA as well as mutations in patient liquid biopsy samples.

Hence, although a variety of rapid assays and strategies have been devised for in vitro diagnostics of emerging pandemias such as Zika virus infections, their implementation typically involves the presence of highly developed health care and laboratory structures. An analyzer device which allows to perform such assays in a simple and low-cost manner has, however, not yet been developed.

There is accordingly a need for low-budget, easy-to-operate and easy-to-produce analyzer devices which are suitable for the performance of complex biochemical assays in less developed environments or situations where high tech infrastructure is missing.

### SUMMARY

The present invention addresses this need and provides an analyzer device for analyzing a sample comprising (i) at least two nested recursive structure elements; (ii) wherein each of the recursive structure elements has at least one connection to at least one of the other recursive structure elements; and (iii) at least one measurement functionality; wherein said analyzer device is configured to allow, via said connections, a displacement of the sample and of material present in a recursive structure element, such as liquid material, to the next recursive structure elements by gravity in the form of a rotation of the analyzer device in a 3D-coordinate system or, optionally, with additional forces such as capillary motion or air or liquid pressure, wherein at least one of the recursive structure elements comprises at least one opening at the surface, configured to receive and/or release the sample for analyzing, wherein the recursive structure elements are suitable to hold liquids within, wherein all recursive structure elements are part of a fluid communication system providing direct or indirect connections between said recursive structure elements, wherein the analyzer device comprises within at least one recursive structure element or in separate recursive structure elements a measuring zone implementing the measurement functionality; and wherein said connections have the form of straight or curved elongations with two openings.

The presented analyzer device advantageously allows to perform complex assays such as CRISPR/Cas-based analyses by moving a sample between different reaction zones, which are organized in a recursive manner, by simple rotation. The analyzer can hence be operated by almost anyone and does not require any profound pre-usage training. The sample to be analyzed can, moreover, be a sample which is directly obtained from a patient on the spot, e.g. a whole blood sample. This renders the device largely independent of typical laboratory infrastructure such as sample logistics and management, external power sources, complicated operational procedures, intensive training programs and allows for in situ analyses at almost any place on earth. In addition, not only the operation of the device is simplified, also its manufacture, since the present invention envisages a production process based on 3D-printing, which can be performed outside of typical factory facilities in a decentralized manner, including in less developed environments. Moreover, this production process allows for a rapid and fast supply of analyzers exactly where they are needed. In addition, the envisaged analyzer device will significantly reduce efforts and time required for the release process for new test assays at the manufacturer's site, and will drastically simplify purchasing and logistic processes at a clinical site. Accordingly, complex bureaucratic release processes for an analyzer and its assays can advantageously be streamlined; and the extensive investment and purchasing procedure can be decreased to a minimum.

According to another embodiment of the present invention, the recursive structure elements, preferably all recursive structure elements, are connected by at least one chute comprising an opening at the surface of the device, configured to receive a cartridge comprising one or more reagents in the form of a reagent pack, and/or one or more samples.

In a further embodiment, said opening is sealable. It is preferred that the opening is a valve, a shutter, a lid, a plug or an adhesive tape. The plastic material is preferably thermoplastic, a thermosetting plastic or a light-activated resin.

In yet another embodiment of the present invention, the recursive structure element comprises, essentially consists of or consists of material, which is printable by a 3D-printer, preferably a plastic or metal material.

Further envisaged is that the direct or indirect connections between said recursive structure elements are closable in a controlled manner.

In further embodiments, the sample for analyzing is an inorganic or organic liquid sample. It is preferred that the sample is a body fluid. Particularly preferred is the employment of a whole blood sample, a blood serum sample, a blood plasma sample, a sputum sample or a urine sample.

In a further embodiment, the form of the analyzer device is a three- to ten-sided prism, a cylinder or a cuboid.

In another embodiment of the present invention, the analyzer device comprises within at least one recursive structure element or in separate recursive structure elements a reaction zone, or a reservoir zone, or combinations thereof.

In a further embodiment the analyzer device as defined above additionally comprises within at least one recursive structure element or in separate recursive structure elements, or in connection with at least one recursive structure element: a measuring unit implementing the measurement functionality, an energy source such as a battery or an accumulator, a heating unit, a cooling unit, a timer unit, a sterilization unit, a magnetic stirrer, a vacuum system, an observation window, a gas inlet, and/or gas outlets.

In yet another embodiment of the present invention, said measuring unit as mentioned above is a magnetometer or a gyroscope sensor configured to determine the change in position of the analyzer device; a hygrometer configured to determine the humidity inside or outside of the analyzer device; a temperature measuring unit configured to determine the temperature at various positions in or on the analyzer device, preferably an infrared sensor, a quartz resonator or a platinum measuring resistor or a silicon bandgap temperature sensor; a photometer configured to detect colors and/or color changes; pH-meter; an electric conductivity measuring unit; a Raman spectrometer or IR spectrometer configured to analyze the composition or particle size of the sample; a viscosimeter; a magnetic resonance imaging scanner; an X-ray scanner; a nucleic acid hybrid analyzer or an ultrasonic sensor configured to determine the volume of the sample.

The present invention further envisages embodiments, wherein recursive structure elements constitute a diagnostic test system, which comprises reagents to perform a diagnostic analysis such as a buffer, a peptide, a protein, an enzyme, a nucleotide, an antibody, a dye, and a measuring unit to detect biochemical reaction results, preferably a photometer configured to detect colors and/or color changes.

In a specific embodiment, the test system is a CRISPR/Cas test system, wherein said test system is a CRISPR/Cas test system, comprising at least: (i) an optional first recursive structure element or reaction zone within a recursive structure element which comprises a reverse transcriptase activity, a buffer and nucleotides; (ii) a second, preferably adjacent, recursive structure element or reaction zone within a recursive structure element which comprises a recombinase, single-stranded DNA-binding proteins (SSBs), and a strand displacing polymerase, a buffer for RPA and nucleotides; (iii) a third, preferably adjacent recursive structure element or reaction zone within a recursive structure element which comprises a transcriptase, preferably a T7 transcriptase, a suitable buffer and nucleotides; and (iv) a fourth, preferably adjacent, recursive structure element or reaction zone within a recursive structure element which comprises an activated RNA binding enzyme of the Cas protein family, preferably a Cas13a enzyme, one or more RNA reporters, preferably an RNA reporter which comprises a dye and a quencher element, and a buffer; and optionally a measuring unit, preferably a fluorometer.

It is further envisaged, in additional embodiments, that one or more of the recursive structure elements comprise a microcontroller.

In yet another set of embodiments, the microcontroller controls an optical and/or audible warning system programmed for indicating the quality and/or correctness of one or more of the following parameters: temperature, pH, electric conductivity, humidity, position of the analyzer device, sequence of operations, movement of samples, direction of movement of the device, emptying and/or filling status of a recursive structure element, blockage of connections between recursive structure elements or external mechanical impact during operation.

In another embodiment, the analyzer device of the present invention rotates manually or automatically, preferably via a rotating element connected on at least one side of the analyzer device, or by a roller element, in a 3D-coordinate system.

In a further aspect, the present invention relates to a method for producing an analyzer device according to the invention, wherein said method comprises 3D-printing of the nested recursive structure elements of the analyzer device and the direct or indirect connections between said recursive structure elements, wherein the recursive structure elements are suitable to hold liquids within, wherein all recursive structure elements are part of a fluid communication system providing direct or indirect connections between said recursive structure elements, wherein the printing process is interrupted at least once to introduce a prefigured measuring unit and optionally one or more prefigured parts of the analyzer device such as a microcontroller, a reagent, a peptide, a protein, an enzyme, an antibody, a composition, an energy source, a heating unit, a cooling unit, a timer unit, a sterilization unit, and/or a magnetic stirrer; wherein said connections have the form of straight or curved elongations with two openings.

In another aspect the present invention relates to a system comprising the analyzer device according to the present invention and at least one reagent pack, wherein the reagent pack is provided as cartridge. The cartridge may preferably comprise a buffer, an acid, a base, a peptide, a protein, an enzyme, an antibody, and/or a dye.

In yet another aspect, the present invention relates to a method for in vitro diagnosis of a viral or bacterial infection or of cancer comprising the diagnostic analysis of a sample in an analyzing device according to the invention or a system comprising the analyzer and a reaction pack of the present invention.

In an embodiment of the method, the method comprises one or more steps of (i) purifying the sample before analysis; (ii) performing a reaction with the sample in one or more recursive structure element according to the invention; and (iii) detecting reaction results preferably by observation in a window or via a measuring unit.

In yet another aspect the present invention relates to a use of the analyzing device or the system of the present invention for the diagnostic analysis of a sample.

In a specific embodiment, said diagnostic analysis is the CRISPR/Cas-based diagnosis of a viral or bacterial infection or of cancer.

It is to be understood that the features mentioned above and those yet to be explained below may be used not only in the respective combinations indicated, but also in other combinations or in isolation without departing from the scope of the invention.

### BRIEF DESCRIPTION OF THE DRAWINGS

FIG. 1 shows a schematic illustration of an embodiment of an analyzer device 100 according to the present invention, comprising, inter alia, recursive structure elements 1, 2, 3, 4 and 5 and an entry opening 16. Also shown are reagents 6, 7 and 8.
FIG. 2 shows a schematic illustration of a further embodiment of an analyzer device 100 according to the present invention, comprising, inter alia, recursive structure elements 1, 2, 3, 4 and 5 and entry openings 16 and 19. Also shown are closable connections between the recursive structure elements and at the entry openings 20 to 26.
FIG.3 schematically depicts another embodiment of an analyzer device 100 according to the present invention comprising, inter alia, recursive structure elements 1, 2, 3, 4 and 5. Also shown are a reservoir zone 31, a measuring zone 32, an observation window 33, a heatable reaction zone 34, a second reservoir zone 35 and a gas inlet/outlet 36.
FIG. 4 shows a further embodiment of an analyzer device 100 according to the present invention comprising, inter alia, recursive structure elements 1, 2, 3, 4 and 5. Also shown are reagents 6, 7 and 8 and receptacle 15 which is provided in measuring chamber 10. The device is placed on an external magnetic field inducer 27.
FIG. 5 shows another embodiment of an analyzer device 100 according to the present invention. The analyzer device comprises a chute 52, which provides interaction zones 53 to 56 for the release of reagents or samples introduced via a cartridge into the analyzer device.
FIG. 6 depicts a similar analyzer device 100 as shown in Fig. 5. The analyzer device comprises a chute 61 which reaches recursive structure element 1, providing interaction zones 62 to 65 for the release of reagents or samples introduced via a cartridge into the analyzer device.
FIG. 7 shows an analyzer device 100 according to the present invention in a rolling device 101. The analyzer device comprises an attachment point of rotation on the left hand side 71 and an attachment point of rotation on the right hand side 72.
FIG. 8 shows a vacuum chamber 81 comprising analyzer device 100. Connected to vacuum chamber outlet 83 is a vacuum pump 84.
Fig. 9 depicts a diagnostic approach for the detection of specific nucleic acids. Starting with a liquid sample 91, double stranded nucleic acids 92 and 93 are generated 110. Subsequently, via the use of a Cas enzyme 95 and its guide RNA 94, a binding to the specific double stranded nucleic acid target becomes possible. Subsequently, nearby inactive reporter molecules 96 are cleft by the Cas enzyme complex 99 which activates the reporter 98.
Fig. 10 shows a similar approach as Fig. 9. Instead of double-stranded nucleic acids here single stranded nucleic acids 112 and 113 are used for the detection procedure.

### DETAILED DESCRIPTION OF EMBODIMENTS

Although the present invention will be described with respect to particular embodiments, this description is not to be construed in a limiting sense.

Before describing in detail exemplary embodiments of the present invention, definitions important for understanding the present invention are given.

As used in this specification and in the appended claims, the singular forms of "a" and "an" also include the respective plurals unless the context clearly dictates otherwise.

In the context of the present invention, the terms "about" and "approximately" denote an interval of accuracy that a person skilled in the art will understand to still ensure the technical effect of the feature in question. The term typically indicates a deviation from the indicated numerical value of ±20 %, preferably ±15 %, more preferably ±10 %, and even more preferably ±5 %.

It is to be understood that the term "comprising" is not limiting. For the purposes of the present invention the term "consisting of" or "essentially consisting of" is considered to be a preferred embodiment of the term "comprising of". If hereinafter a group is defined to comprise at least a certain number of embodiments, this is meant to also encompass a group which preferably consists of these embodiments only.

Furthermore, the terms "(i)", "(ii)", "(iii)" or "(a)", "(b)", "(c)", "(d)", or "first", "second", "third" etc. and the like in the description or in the claims, are used for distinguishing between similar elements and not necessarily for describing a sequential or chronological order.

It is to be understood that the terms so used are interchangeable under appropriate circumstances and that the embodiments of the invention described herein are capable of operation in other sequences than described or illustrated herein. In case the terms relate to steps of a method, procedure or use there is no time or time interval coherence between the steps, i.e. the steps may be carried out simultaneously or there may be time intervals of seconds, minutes, hours, days, weeks etc. between such steps, unless otherwise indicated.

It is to be understood that this invention is not limited to the particular methodology, protocols etc. described herein as these may vary. It is also to be understood that the terminology used herein is for the purpose of describing particular embodiments only, and is not intended to limit the scope of the present invention that will be limited only by the appended claims.

The drawings are to be regarded as being schematic representations and elements illustrated in the drawings are not necessarily shown to scale. Rather, the various elements are represented such that their function and general purpose become apparent to a person skilled in the art.

Unless defined otherwise, all technical and scientific terms used herein have the same meanings as commonly understood by one of ordinary skill in the art.

As has been set out above, the present invention concerns in one aspect an analyzer device for analyzing a sample comprising (i) at least two nested recursive structure elements; (ii) wherein each of the recursive structure elements has at least one connection to at least one of the other recursive structure elements; and (iii) at least one measurement functionality; wherein said analyzer device is configured to allow, via said connections, a displacement of the sample and of material present in a recursive structure element, such as liquid material, to the next recursive structure elements by gravity in the form of a rotation of the analyzer device in a 3D-coordinate system, or, optionally, with additional forces such as capillary motion or air or liquid pressure, wherein at least one of the recursive structure elements comprises at least one opening at the surface, configured to receive and/or release the sample for analyzing, wherein the recursive structure elements are suitable to hold liquids within, wherein all recursive structure elements are part of a fluid communication system providing direct or indirect connections between said recursive structure elements, wherein the analyzer device comprises within at least one recursive structure element or in separate recursive structure elements a measuring zone implementing the measurement functionality; and wherein said connections have the form of straight or curved elongations with two openings.

As used herein the term "recursive structure elements" relates to structure elements which are defined in terms of their type. The structure is thus, when starting at a smallest or innermost section, followed by the same type of structure, albeit in a larger dimension; or vice versa, when starting from a largest or outermost section, the structure is followed by the same type of structure, albeit in a smaller dimension. The recursive structure elements according to the present invention are hence provided in some analogy to Russian dolls or Matryoshka dolls, i.e. as set of wooden dolls of decreasing size place one inside another, which have a recursive structure since the same type of structure is repeated at every instance from large to small.

Thus the recursive structure elements are provided in a nested, preferably in a symmetrically nested form.

The number of recursive structure elements present in an analyzer device according to the present invention is not limited. The number may be, for example, 2, 3, 4, 5, 6, 7, 8, 9, 10, 11, 12, 13, 14, 15, 16, 17, 18, 19, 20 or more than 20 recursive structure elements within an analyzer device. In specific embodiments, the recursive structure elements may further be combined or associated with non-recursive structure elements. Such non-recursive structure elements may, for example, be specific chamber elements, reservoirs, inlets, outlets, tubings or functional units etc. at different locations within the device, e.g. at an outermost layer, or an innermost layer. The recursive structure elements may have any suitable form. For example, the element may have, when observed in top view and/or side view, a quadratic form with identical or similar sides, or with differently sized sides, e.g. in the form of a rectangle. Also envisaged are further different forms such as triangles, circles, trapezoids, pentagons, hexagons, heptagons, octagons, nonagons, decagons etc. or any other suitable polygon or any combination thereof; it may also have non-symmetrical or variable side proportions. When observed as three-dimensional entities, the recursive structure elements may have, for example, the form of a prism starting with a polygon as described above, or of cylinder or roll. In further embodiments, the structure may be a symmetrical polyhedron such as a tetrahedron, cube, octahedron, dodecahedron or icosahedron. It is particularly preferred that all or almost all, e.g. about 99%, 95%, 90%, 85% or 80% of all recursive structure elements recursive structure elements of an analyzer device according to the present invention have the same form, yet in a different dimension. In further specific embodiments, also one or more of the above mentioned forms may be combined, i.e. different recursive structure elements may have one or more different forms, e.g. complementary forms, within one analyzer device. The succession of recursive structure elements within the analyzer may follow any suitable proportion. For example, the next largest recursive structure element, as measured by the length of at least one side, or as measured by the volume, may be 5%, 7.5%, 10%, 15%, 20%, 25%, 30%, 40%, 50%, 60%, 70%, 80%, 90%, 100%, 125%, 150%, 200%, 300%, 400%, 500% or more or any value in between the indicated values larger than the enclosed or next smaller recursive structure element. In one embodiment, all recursive structure elements in a device may show the same size or volume increment as described above. In other embodiments, the size or volume increment may be different, e.g. from first to second recursive structure element 20%, from second to third recursive structure element 30 % etc., or vice versa.

The overall form of the analyzer device according to the present invention may be any suitable form allowing for a rapid and easy manufacture, handling, storage and transport of the device. The overall form may, in certain embodiments, follow the form of the recursive structure elements, e.g. as defined above. For example, if all or almost all of the recursive structure elements of an analyzer device, e.g. about 99%, 95%, 90%, 85% or 80% of all recursive structure elements have the form of a prism starting with a polygon or have the form of a cylinder as described above, the analyzer device comprising said recursive element structures may also have the form of a prism staring with a polygon or have the form of a cylinder as described above. Similarly, if all or almost all of the recursive structure elements of an analyzer device, e.g. about 99%, 95%, 90%, 85% or 80% of all recursive structure elements have the form of a symmetrical polyhedron such as a tetrahedron, cube, octahedron, dodecahedron or icosahedron as described above, the analyzer device comprising said recursive element structures may also have the form of a symmetrical polyhedron such as a tetrahedron, cube, octahedron, dodecahedron or icosahedron as described above. In certain embodiments of the invention, the analyzer device may have the form of a three- to ten-sided prism, the form of a cylinder, roll or of a cuboid. The analyzer device may further comprise additional functional elements at the outside which allow for movement via external apparatuses. For example, the analyzer device may comprise one or more, preferably two, attachment points of rotation on a side, e.g. on the left and right side, or at the front and rear side etc. It is preferred that in case two or more attachment points are present these attachment points are at opposite sides of the analyzer device.

The size or volume of the recursive structure element is not specifically limited. In certain embodiments, the volume of a recursive structure element may be at least 0.1 ml, at least 0.5 ml or at least 1.0 ml. In further embodiments, the volume of a recursive structure element may be at most 10 ml, at most 25 ml, at most 50 ml, at most 100 ml, at most 250 ml, at most 500 ml, at most 1000 ml or at most 2000 ml, or any value in in between the lower and upper limits mentioned above. In further embodiments, the overall dimension of the analyzer device may not be particularly limited. The dimensions may follow the dimensions or volume of the recursive structure element. Furthermore, the dimensions may be dictated or influenced by the production process for the analyzer, e.g. the 3D printing approach, the 3D printer assembly etc.

According to certain embodiments, at least some of the recursive structure elements according to the present invention are envisaged to fulfill the function of chambers allowing for the contacting of a sample with a reagent, or for the performance of an analysis of a sample. Generally, the recursive structure element is suitable to hold liquids within. Within an analyzer device at least two of the recursive structure elements comprised are suitable to hold liquids within.

The recursive structure element according to the invention may be provided with one or more reaction zones which are capable of holding reagents and/or samples. The material is accordingly hold at the specific reaction zone without flow of the material out of the zone, or at least out of the recursive structure element. A reaction zone may, for example, be located at each side of the recursive structure element. Alternatively, the recursive structure element may be portioned into bottom, wall and ceiling sections, wherein each section may have a different function or provide different reagents etc. For example, the reaction zone may be provided only at the bottom, or only at the bottom and one two, or three wall sections. In further embodiments of the present invention, the recursive structure element may comprise 2, 3, 4, 5, 6, 7, 8, 9, 10 or more sub-zones. These sub-zones may spatially be separated or have a compartment structure, e.g. are separated by separative elements. In preferred embodiments, reagents may be immobilized at one or more of the corresponding reaction zones.

In further embodiments, a recursive structure element may comprise a reservoir zone. A reservoir zone may, for example, be filled with water, buffers, reagents, dyes, gas etc. or constitute a dumping or intermediate storage place for samples or materials during a displacement move. The recursive structure element may, in specific embodiments, be entirely in the form of a reservoir zone, or comprise one or more reservoir zones, e.g. in the form of sub-zones, which may spatially be separated or have a compartment structure, e.g. are separated by separative elements. A reservoir zone may, in preferred embodiments, be connected to other zones or, if the entire recursive structure element is a reservoir, to other recursive structure elements by controllable connections which can be opened or closed according to necessity, e.g. when a certain reagent, buffer, dye etc. is required for a reaction, if a certain parameter is outside of a predefined range and has to be changed, if a reaction has to be stopped by dumping the material etc.

In further embodiments, a recursive structure element may comprise a measuring zone. This measuring zone may implement a measurement functionality as defined herein. The measuring zone may accordingly be equipped with elements allowing for the measurement of specific parameter, e.g. pH, temperature, color, change of color, electric conductivity etc. A measuring zone may be present in each recursive structure elements, or be present in only one or a few recursive structure elements within an analyzer device. Also envisaged is the presence of one or more measuring sub-zones. These sub-zones may spatially be separated or have a compartment structure, e.g. are separated by separative elements.

Accordingly, an analyzer device according to the present invention may comprise, within at least one recursive structure element, the above mentioned reaction zone, reservoir zone and/or measuring zone. It is preferred that the analyzer device comprises said zones in separate recursive structure elements, or that some zones are provided in specialized recursive structure elements, e.g. measuring zones or reservoir zones, whereas other zones such as reaction zones may be present in more than one, e.g. the majority of recursive structure elements within an analyzer device.

Within an analyzer device according to the present invention, each of the recursive structure elements has at least one connection to at least one of the other recursive elements. The connection may, in a simple but non-limiting example, be in a sequential or serial order from the innermost or smallest recursive structure element to the next largest recursive structure element and so forth. Also non-sequential or non-serial orders are envisaged. In further embodiments, there may be more than one connection between two recursive structure elements, e.g. 2, 3, 4, 5 or more. The accordingly connected recursive structure element may be the next largest/next smallest recursive structure elements. In yet another embodiment, the connections may be present also between any non-adjacent recursive structure elements or levels or recursive structure elements, e.g. from the outermost recursive structure element to the innermost recursive structure element etc. In this embodiment the number of connections may be one or more than one. The number of connections may further vary between the different recursive structure elements in a device. The connection may be implemented by the presence of a tubing, a channel, a capillary channel, a fluid flow channel, a bridge or any other suitable connecting element. The connection is envisaged to allow material to enter or leave a recursive structure element by gravity, capillary motion or on the basis of any suitable additional force such as air or liquid pressure. It is preferred that the connections allow the material to enter or leave a recursive structure element in a minimum of time, thus reducing to the residency time in the connections to a negligible amount. The connections of the analyzer according to the invention have the form of straight or curved elongations with two openings. The size of the connections may vary with the distance between the recursive structure elements which they connect. For example, the connection may, in one embodiment, be simple apertures or openings in the wall, which connect two adjacent recursive structure elements. The position of the connections or openings in a recursive structure element may be adapted to the intended use. Typically, the connection or opening is located in a sector of the wall which is remote from the zone where the material reacts or where it arrives after a displacement move. This may, for example, be close to the ceiling section, if the material arrives first or is supposed to react at a bottom section, or vice versa. Such a location allows the material or sample to leave the recursive structure element only after a further rotational movement.

In a specific embodiment of the present the recursive structure elements are part of a fluid communication system providing direct or indirect connections between the recursive structure elements. The term "fluid communication system" as used herein means that liquids, e.g. a sample or material in the device, can be transported or displaced from one recursive structure element to at least a second recursive structure element. In certain embodiments, the transport may be performed through 2, 3, 4, 5, 6, 7, 8, 9, 10 or more recursive structure elements. In further embodiments, all, almost all, e.g. about 99%, 95%, 90%, 85% or 80% of all recursive structure elements of a device, or at least half of the recursive structure elements of a device form part of the fluid communication system. A "direct connection" as used herein refers to a fluid communication between two adjacent recursive structure elements. An "indirect connection" as used herein refers to a fluid communication between two recursive structure elements via at least one additional recursive structure element between said two recursive structure elements.

It is further preferred that the connections within said fluid communication system are closable and/or openable in a controlled manner. The control may, for example, be exerted by rotational moves as described herein, or by electronically controlling or radio controlling the opening/closing status of connections. By providing such a control mechanism, certain sectors of the device may be separated from the rest of the device, streams of liquid my be directed towards specific sectors of the device and/or reagents etc. may be added or introduced into reaction zones according to demand.

Any opening or connection may accordingly, in specific embodiments, be equipped with a seal, lock or latch, allowing the material to be separated between two recursive structure elements or controlling the movement material and the reachability of certain recursive structure elements.

The displacement of a sample between the connected recursive structure elements can be achieved by a rotation of the analyzer device in a 3D-coordinate system. The displacement may, in specific embodiments, displace, in addition to the sample, any material, preferably liquid material, present in a recursive structure element, to the next recursive structure element or the outside of the device. By relying on a rotational movement, gravity is mostly used to displace the sample or material. In certain embodiments, additional forces such as capillary motion or air or liquid pressure may be used. For example, after the rotation has been performed an air or liquid stream may be used to fully empty a recursive structure element and/or the connections between the recursive structure elements. In other embodiments, one or more capillary channels may be present, which do not require gravity for displacement. In such a scenario, a rotational move may bring the material to the entry point of a capillary channel which then, based on capillary motion, displaces the material.

The rotational movement in the 3D-coordinate system may be performed in any suitable direction, e.g. forward or backwards, or left or right, as well as any inclination between these principle directional movements. The angle which is covered by one rotational step in the 3D coordinate system may have any suitable value. Typically, the angle corresponds to the position of the opening/connections between two connected recursive structure elements. In a preferred example, the covered angle of one rotational step in the 3D coordinate system is 45°, 60°, 90°, 120°, or 180°. It is particularly preferred that the angle is 90° or 180°. Any suitable sequence of rotational moves may be implemented, e.g. first forward, then left, then backwards, then right, which can be repeated one to several times etc.; or first right, then backwards, then left, then forwards which can be repeated one to several times etc.; or first left, then right, then left, then forwards, which can be repeated one to several times etc.; or any further combination of moves and repetition of moves. The rotational moves may, in certain embodiments, be implemented as manually performed rotations. The sequence of rotational moves may, in further embodiments, be indicated in an information leaflet, instruction video or data file to the user, i.e. the leaflet, instruction video or data file describes the sequence of moves to be performed and shows how the device has to be rotated manually in order to displace the sample and/or material comprised in it. In further embodiments, the analyzer device may be equipped with an electronic indication system explaining the next rotational move to the user. In addition, checking, controlling and alarming elements may be provided which inform the user about wrong or incorrect or not finished rotational moves.

In further, alternative embodiments, the rotational moves of the analyzer may be implemented as automatically performed rotations. The automatically performed movements may be implemented via a rotation element which is connected to at least one side of the analyzer device. Another non-limiting example of a suitable automatic rotation system, is a roller element. These automatic systems may be adapted to the form and function of the analyzer device. For example, a roller element may preferably be used with a cylindric analyzer device. Cuboid analyzer devices may be rotated with the help of connected rotation elements at both sides, left and right, or top and bottom, of a cuboid structure. The automatic rotation may accordingly be performed with any suitable rolling device which preferably comprises a controlling mechanism to define the rotational velocity, the direction and number of rotational moves, possible time intervals between rotational moves etc. It may, in a simple embodiment, be equipped with a motor, a microcontroller, a user interface and a receptacle for the analyzer device or connector where the analyzer device can be attached.

In certain embodiments, the connections or openings between recursive structure elements, e.g. two connected recursive structure elements, may be closed and/or opened in accordance with the reactions to take place. For example, only after a reaction has been finished, the connection to the next recursive structure element is possible by specifically opening said connection, e.g. after a certain period of time or in reaction to an external signal or induced by the next rotational movement.

The analyzer device according to the present invention additionally comprises at least one measurement functionality. The term "measurement functionality" as used herein relates to any entity, instrument, unit, compound or composition which allows to determine the status, consistency or quality of a sample; to determine the presence or absence of a specific compound, particle, nucleic acid, protein, structure etc. in a sample; to determine the result of a reaction performed on or with the sample; or to determine any parameter connected to the operation, status, performance, handling etc. of the device itself. Such a measurement functionality may, for example, allow to determine the change of color, or the color homogeneity if a reaction with a dye has been performed. It may, in another example, allow to determine the pH, electric conductivity, turbidity or cloudiness in a solution, or the temperature or any other parameter which is involved in a reaction performed.

Furthermore, the analyzer device may be provided in the form of an aggregate, group or array of several analyzer devices. Within such an aggregate, group or array of analyzer devices, the devices may have identical, similar or different forms and functions. Accordingly, the number of recursive structure elements of devices within said groups may vary. In further embodiments, the devices within an aggregate, group of array of devices may be connected, e.g. in an adjacent or sequential format, or in a centralized or semi-centralized format, or any combination of these formats

In further embodiments at least one of the recursive structure element may comprise an opening at the surface. This opening is typically configured to receive a sample for analyzing. In specific embodiments, the opening may also receive additional material such as reagents or water. The opening may, for example, be a fluid flow channel, which may optionally be equipped with air or liquid stream displacement functionality, or a capillary channel, which is designed to receive a liquid sample such as a blood droplet. By gravity or capillary motion or any other external force, the sample material may be displaced to a first recursive structure element. The opening may connect any suitable recursive structure element with the surface, e.g. an innermost recursive structure element or an outermost recursive structure element. Further envisaged is the presence of more than one opening, e.g. 2, 3, 4, 5, 6, 7, 8, 9 or 10 or more openings. These openings may be connected with one recursive structure element which can, for example, be equipped with specific sub-zones, e.g. as defined above, receiving each sample from each opening separately. Alternatively, the openings may be connected with different recursive structure elements. Such a design may advantageously be used for a parallel sample analysis.

In further embodiments, the opening may be configured to release a sample from the device. The opening may, in further embodiments, also be configured to release additional material such as reagents etc. from the analyzer. An opening configured to release a sample or material from the device is typically connected to a different recursive structure element then the opening for receiving a sample. The connection may have the same form or function as described above for the receiving opening.

In another embodiment, two or more of the recursive structure elements are connected by at least one chute comprising an opening at the surface of the device. It is particularly preferred that all or almost all of the recursive structure elements, e.g. about 99%, 95%, 90%, 80%, 70%, or 60% of the recursive structure elements are connected by said chute. The nested structure of the recursive structure elements in the device accordingly allow the provision of a single chute which reaches every recursive structure element. The chute may advantageously be used to enter a cartridge or similar element from the outside to the device. Such cartridge may, for example, comprise one or more reagents, buffers, reaction terminators, dyes, enzymes etc., and/or one or more measurement functionalities, e.g. measurement units which can at once be inserted into the device. For example, the cartridge may comprise a reagent pack prepared for the analysis of a specific target in a sample, e.g. a specific disease or infection. It may hence comprise necessary reagents and ingredients and/or measurement functionalities, e.g. in the form of measurement units adapted to the specific analysis purpose. Thus, by providing different cartridges for an analyzer device, a multitude of different analyses becomes possible on the basis of a single analyzer device form or design.

By placing the chute at a central or near-central position of the device, all recursive structure elements may be reached. In certain embodiments, it is envisaged that not all recursive structure elements may be reached by the chute, or that they are sealed against the chute, e.g. via a wall, since one or more recursive structure elements may have different functions and accordingly not require the presence of externally introduced reagents or units. In further embodiments, the chute may reach the recursive structure elements once. In other embodiments, the chute may reach the recursive elements more than one time, e.g. if the chute is designed to span the device from a top surface to a bottom surface, or from a rear to a front surface, or from one point of the surface to the opposite point at the surface. If a second or further connection with the chute and/or the introduced cartridge is not intended, the recursive structure element may be sealed against the chute, e.g. via a wall or insulator structure, which may optionally be provided by an introduced cartridge.

In another set of embodiments, the chute may be configured to receive one or more samples. The samples may accordingly be provided on shaft or cartridge element. For example, 1, 2, 3, 4, 5, 6, 7, 8, 9, 10 or more samples may be placed on receptacle cartridge, e.g. by taking the sample directly or by, for example, pipetting the sample into the cartridge. Subsequently, the cartridge comprising the samples may be introduced into the chute and thus be provided to one or more recursive structure elements at the same time.

Also envisaged is the provision of cartridges which allow both, the introduction of samples and reagents via a chute.

Further envisaged by the present invention is the presence of more than one chute in an analyzer device. For example, two chutes may be present, one chute configured to receive a reagent pack or a cartridge comprising reagents as described above, and second chute configured to receive a sample as described above. Also contemplated is the presence of two or more chutes for the introduction of cartridges providing different reagents, or one cartridge providing reagents and another one providing one or more measurement units.

In further embodiments, an opening or chute as described above may be sealed. The sealing shall prevent any draining or spilling of sample or material from the device. It may further prevent dropping out of a cartridge or reagent pack which has been inserted into a chute as described above. The sealing may have any suitable form, which can be adapted to the form and function of the opening. The sealing may be reusable, e.g. have the form of a valve, shutter, lid, plug or latch which can be closed and opened several times. Alternatively, the sealing may be a single use element, which cannot be reopened and/or reclosed a second or further time. For example, the sealing may be provided by an adhesive tape, or by applying curing glue, a 2-component glue, a plastic being hardened by ultra-violet-light, a hot melt adhesive, a sealing wax or similar substances which close the opening in a waterproof manner. In further embodiments, the opening may be closed in an airproof or gas-proof manner.

The material of the recursive structure elements or of other parts of the analyzer device may be any material suitable for the performance of molecular analyses, preferably of diagnostic analyses. It is preferred that the material is chemically or biochemically inert, non-toxic and resistant towards torsions or tensions, towards high or low temperatures, high or low pH and/or towards UV or radioactive radiation. The material may be comprised of a single layer, or may comprise two or more layers. If two or more layers are present, a basic layer may, for example, be covered by a coating. Accordingly, some of the qualities of the material, e.g. being chemically or biochemically inert and/or non-toxic may be provided by the coating layer only. In further embodiments, the material may be functionalized. It may, for example, comprise protrusions, edges or zones of roughness, which facilitate molecular interactions. Furthermore, the material may comprise areas offering certain functions, e.g. by immobilization of elements such as antibodies, lectins, enzymes, inorganic catalyst etc. Also envisaged is the provision of functions via magnetic properties, e.g. the presence of magnetic zones which allow the pulling down of ferromagnetic particles etc., or the provision of a zone which allows the usage of magnetic stirrers. Further envisaged is the presence of functionalized material allowing for inductive heating or manipulation of air or liquid streams.

In accordance with preferred embodiments of the present invention, the recursive structure element comprises, essentially consists of or consists of material which is printable by a 3D-printer. Advantageously, it is contemplated to provide the analyzer device of the present invention as 3D printing product. It is hence easy and rapid to produce, does not require any sophisticated manufacturing facility and can be obtained with simple and easy to obtain ingredients. Moreover, by relying on 3D printing, there is no necessity to transport the device to remote and difficult to reach places.

This allows for an extremely fast provision of the analyzer device even in very difficult to reach regions.

In preferred embodiments, the recursive structure elements as described above are produced by a 3D printing procedure. A 3D printing procedure is typically performed by specialized 3D printers which may, in typical embodiments, comprise a controllable printer head and a substrate onto which material can be printed. Examples of suitable 3D printers would be known to the skilled person or can be derived from internet resources such as https://www.3dnatives.com or similar sites. The printer head is typically moveable relative to the substrate in a 3 axis motion (i.e. x, y and z axis, where x and y represent the horizontal motion, and z the vertical motion). In typical embodiments, the printer head and/or the substrate are moveable. The term "3D printing" thus relates to the step of delivering material from a printer head to the vicinity of the substrate. In the initial stages of the printing process the material may be delivered onto the substrate surface, thereby forming a layer of material on that surface. Subsequently, the material may be delivered onto a previously deposited layer, thereby forming another layer of material. After the termination of the printing procedure, the printed device can be removed from the substrate. The substrate may, in specific embodiments, be a paper substrate, or form part of the final structure of the device.

The printing procedure typically makes use of plastic or metal precursor material to produce the recursive structure elements of the analyzer device of the present invention. Accordingly, the printing procedure may make use, and the printed product accordingly comprise, essentially consists of or consists of thermoplastic, thermosetting plastic, light-activated resin or metal material. In further embodiments, the material of the device, i.e. of the recursive structure elements, may be derived from a precursor material, which is printable from a 3D printer. Suitable precursor material may further be converted into the material of the device, i.e. the recursive structure elements, after printing, e.g. via light or UV-curing. In further embodiments, the recursive structure elements may be partially provided by a 3D printing procedure and partially provided in a different form, e.g. introduced into a 3D printed scaffold after or during the printing process.

Examples of suitable thermoplastic materials include, but are not limited to, acrylonitrile butadiene styrene (ABS), polystyrene, polycarbonate, polypropylene, polyvinyl chloride, polyvinyl alcohol (PVA), polyethylene, high-density polyethylene (HDPE), polylactic acid (PLA) and polyamides such as Nylon, cycloolefin copolymer (COC), cellulose acetate, ethylene-vinyl acetate (EVA), fluoroplastics (PTFE, with FEP, PFA, CTFE, ECTFE, ETFE), acrylic/PVC alloys, polyaryletherketone (PAEK), polybutadiene (PBD), polybutylene (PB), polybutylene terephthalate (PBT), polycaprolactone (PCL), polychlorotrifluoroethylene (PCTFE), polyethylene terephthalate (PET), polycyclohexylene dimethylene terephthalate (PCT), polycarbonate (PC), polyhydroxyalkanoates (PHAS), polyketone (PK), polyacrylates, polysulfone (PSU), polytrimethylene as well as polyvinyl acetate.

Thermosetting plastic materials are typically stronger than thermoplastic materials due to the three-dimensional network of bonds and are thus better suited for high-temperature applications. Examples of suitable thermosetting plastic materials include, but are not limited to polyester resins, polyurethans, polyuria/polyurethane hybrids, vulcanized rubber, bakelit, duroplast, urea-formaldehyd, melamine resin, diallyl-phtalate (DPA), epoxy novolac resin, benzoxazines, polyimides and bismaleimides, cyanate esters, furan resins, silicone resins, thiolyte and vinyl ester resins.

Examples of suitable light-activated, e.g. UV-curable, resins include, but are not limited to, light-curing acrylate adhesives, light-curing silicones, light-curing cyanoacrylates, light-curing epoxy resins, and light-curing anaerobic adhesives.

Examples of suitable metals include, but are not limited to, titanium, aluminium, cobalt, chrome, nickel, gold, platinum and stainless steel. Also envisaged are metal alloys, in particular low-melting point alloys, such as alloys of aluminium, titanium, cobalt-chrome superalloys, nickel superalloys, alloys of steel. Further envisaged are composite materials comprising plastics as defined above with metal additives, e.g. materials as offered by color Fabb, ProtoPlasta or TreeD Filaments.

It is particularly preferred that the material is acrylonitrile butadiene styrene.

Further examples of suitable materials would be known to the skilled person or can be derived from suitable literature sources such as Cohen et al., Tissue Engineering, 2006, 12, 1325, Malone et al., Rapid Prototyping Journal 2004, 10, 58 or Nicolas Wirth, Bachelor Thesis "Strategic Assessment of the Potential of the 3D Printing Technology from Siemens Healthcare's Perspective", International Business School, Nürnberg, 2015.

In a corresponding aspect the present invention also relates to a method for producing an analyzer device by 3D-printing of the recursive structure elements of the analyzer device, wherein said method comprises 3D-printing of the nested recursive structure elements of the analyzer device and the direct or indirect connections between said recursive structure elements, wherein the recursive structure elements are suitable to hold liquids within, wherein all recursive structure elements are part of a fluid communication system providing direct or indirect connections between said recursive structure elements, wherein the printing process is interrupted at least once to introduce a prefigured measuring unit and optionally one or more prefigured parts of the analyzer device such as a microcontroller, a reagent, a peptide, a protein, an enzyme, an antibody, a composition, an energy source, a heating unit, a cooling unit, a timer unit, a sterilization unit, and/or a magnetic stirrer; wherein said connections have the form of straight or curved elongations with two openings. The method generally comprises steps of printing successive layers of material according to a printing template.

The method may, in certain embodiments, include the provision of a printing template, e.g. derivable from a scanning approach or produced de novo on the basis of architectural/geometric information about the form and function of the analyzer device of the present invention. The printing template may further be obtained from suitable databases or internet download sites. The method may, in further embodiments, additionally comprise the provision of a suitable programmable 3D-printer. The printer preferably comprises.

The present invention further envisages the provision of a program for controlling a method of 3D-printing the recursive structure elements of the analyzer device of the present invention. The program may be provided, for example, on a memory device such as a disk or flash memory or the like. The program may also be hosted on a website, and it may be downloadable from a database or the internet. The computer may be provided with a user interface to allow a user to control the printing template and thus to control the shape, dimensions etc. of the printed elements. In a further specific embodiment, the program to be used for the printing process may be an open-source program which allows user modifications. It is envisaged by the present invention to use such a program in order to implement a break or pause during the printing process, allowing for the introduction of not printed components as described above. The printing process may afterwards be continued.

Further steps of the 3D printing method include the contacting of the printer nozzle with a reservoir comprising the printable precursor material by fluid communication. Subsequently, the printable material may be delivered from the reservoir to the nozzle as required, and the material may be dispensed from the nozzle to the vicinity of the substrate. The movement of nozzle and the communication with the reservoir may be controlled by an operating system running on an associated computer.

According to the invention the printing activity is interrupted at least once to introduce a prefigured measuring unit and optionally one or more prefigured parts of the analyzer device. Such prefigured, i.e. not 3D printed, parts, include a microcontroller, a reagent, a peptide, a protein, an enzyme, an antibody, a composition, an energy source, a heating unit, a cooling unit, a timer unit, a sterilization unit, and/or a magnetic stirrer. After said parts have been introduced, the printing activity can be resumed. The interruptions may be based on pre-programmed pauses in the printing template.

In alternative embodiments, one or more of the mentioned parts may themselves be 3D printed into or onto a recursive structure element of the present invention. For example, 3D printing may be used to deliver reagents, peptides, proteins, enzymes, antibodies or any suitable composition into or onto one or more recursive structure elements of the present invention. In these embodiments, the printing is performed either with two or more different 3D printers, or the 3D printer is capable of making use of different precursor materials.

After the printing process or part of the printing process is finished, the printed device or part of it may be subjected to finishing operations such as curing, e.g. with light, heating, sterilization, washing and/or drying.

The term "sample" as used herein relates to any inorganic or organic liquid sample. Preferably, the sample is biological sample. The term "biological sample" as used herein refers to any specimen obtained from a biological organism, preferably a living organism. The term relates also to specimen obtained from non-living, i.e. dead biological organisms, in particular recently deceased organisms. The term "biological organism" includes in general eukaryotic systems and may also comprise sub-portions of eukaryotic systems. In particular, such organisms include higher eukaryotes. In preferred embodiments of the present invention a biological sample may be derived from an animal, preferably from a mammal, e.g. from a cat, a dog, a swine, a horse, a cattle, a sheep, a goat, a rabbit, a rat, a mouse, a monkey. Particularly preferred is a sample obtained from a human being.

The sample may be obtained via suitable methods known to the person skilled in the art. The sample used in the context of the present invention should preferably be collected in a clinically acceptable manner, more preferably in a way that nucleic acids (in particular RNA) or proteins are preserved.

The sample may include body tissue as well as feces or stool which have to be pretreated, i.e. liquidized, in order to be usable in the context of the present invention. Preferred are body fluids, such as blood, sweat, sputum or saliva, semen and urine. Particularly preferred are body fluid samples such as whole blood, blood serum, blood plasma, sputum and urine.

In further embodiments the analyzer device additionally comprises within at least one recursive structure element a measuring unit, an energy source, a heating unit, a cooling unit, a timer unit, a sterilization unit, a magnetic stirrer, a vacuum system, an observation window, a gas inlet, or and/or a gas outlets. Also envisaged is any further functional unit which may suitably be used for the performance of molecular analyses such as diagnostic analyses on samples as defined herein. Examples include elements which establish an electric or electrophoretic current, density detection units, flow velocity detection units, unit performing molecular reactions, e.g. PCR reactions or other amplifications, a camera or photographic unit etc.

These additional elements may, in further embodiments be provided in separate recursive structure elements. Accordingly, in one recursive structure element one additional element, i.e. a functional unit as mentioned above, may be present and in another one, e.g. adjacent recursive structure element, a further, different or similar functional unit may be present. It is also contemplated that not all recursive structure elements within one analyzer device comprise such functional units, but only a sub-portion, e.g. about 1%, 5%, 10%, 15%, 20%, 30%, or 40% or any value in between the mentioned values. In a further group of embodiments it is also envisaged that these additional elements are provided outside of recursive structure elements. In these embodiments, the additional elements may be in connection with one, or preferably more than one recursive structure elements. For example, a heating or cooling unit may either be located in one or more specific recursive structure element, or it may be located in a sector of the analyzer device which is connected to two or more recursive structure elements, e.g. in a wall structure between two or more recursive structure element. Further envisaged are additional elements, i.e. functional units as defined herein, which are connected to one or more recursive structure elements via a tubing or fluid communication systems. For example, hot or cool/cold gas, e.g. air or liquid, may be provided to one or more recursive structure elements via tubing systems reaching these one or more recursive structure elements. The tubings may, in specific embodiments, further be closable and/or openable in a controlled manner, e.g. via rotational movements, or electronically etc. Accordingly, one heating or cooling unit may be sufficient to heat or cool two or more recursive structure elements.

An "energy source" as used herein is typically an electric power source. In order to be operational, device may thus have its independent electric power source. This may, for example, be a battery or a rechargeable battery or an accumulator. In further embodiments, the electric power may be provided externally, e.g. by wireless power transfer (WPT) or wireless energy transmission. These technologies use different types of electromagnetic energy, including electric fields, magnetic fields, radio waves, microwaves or infrared waves. In a WPT scenario a transmitter module may be present in the vicinity of the device. This technology may further be used to recharge batteries of in device during recovery periods or before usage.

A "heating unit" as used herein refers to module which allows, within a defined area or zone, to keep the temperature constant at a desired, high value, or where the temperature may be set to a desired, high value, e.g. in dependence on the analysis to be performed. A "cooling unit" as used herein refers to module which allows, within a defined area or zone, to keep the temperature constant at a desired, low value, or where the temperature may be set to a desired, low value, e.g. in dependence on the analysis to be performed. Such areas or zones may further also be equipped with suitable temperature measuring units allowing the measurement of temperature changes or temperature gradients etc.

A "timer unit" as used herein may, for example, be a timer connected to inlet and/or outlet sections of a recursive structural element, thus allowing the determination of the start and/or end time point of a reaction, e.g. determining when the sample has arrived or left the recursive structural element. Furthermore, the timer unit may be connected with a controlling element determining the opening or closing status of connections between recursive structural elements as defined herein. In a specific embodiment, the time may allow the opening and/or closing of said connections, e.g. when a rotational movement is planned.

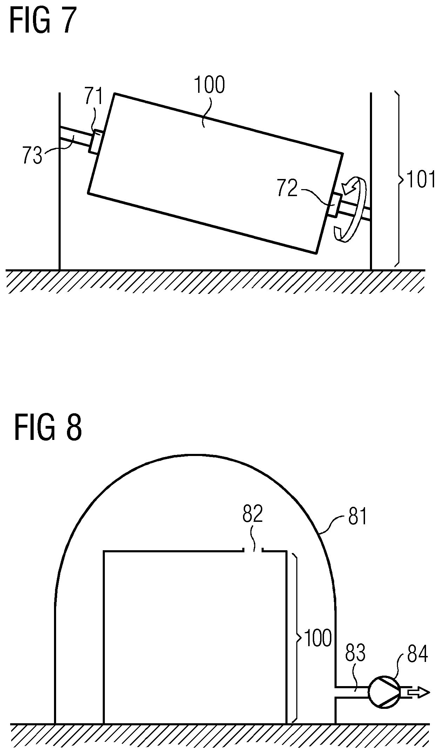

A "sterilization unit" as used herein relates to any aggregate which allows to sterilize at least a part of the analyzer device according to the invention. For example, the sterilization may be performed in one or more recursive structural elements, or in or more zones as defined herein. This sterilization functionality may, for example, be provided by UV radiation, chemical sterilization, air ventilation or heating. Also envisaged is any combination of the above. In further embodiments, the sterilization process is performed with the help of a vacuum chamber, e.g. as depicted in Fig. 8. The vacuum chamber may comprise the analyzer device entirely. The device, which advantageously comprises an opening, may accordingly be evacuated to remove any air borne specimen. In a further preferred embodiment, the vacuum chamber may be used in an attempt to exsiccate the analyzer device. Accordingly, any liquid present in the device may be dried by use of a vacuum chamber.

In a further embodiment a magnetic stirrer is used in the analyzer device of the present invention. The term "magnetic stirrer" as used herein refers to a system wherein, by applying a rotating magnetic field, e.g. from the outside, a magnetic stir bar is spun in the device, thus allowing to stir any liquid or material in which the stir bar is immersed. The magnetic field may be provided, for example, by a rotating magnet or a set of stationary electromagnets placed beneath the section of the device where the stir bar is located. The device may accordingly comprise one or more stir bars, e.g. at different locations allowing stirring in several planes of the 3D coordinate system. The stir bar may be provided in any suitable form or shape, e.g. as sterile, chemically inert entity which does, in further embodiments, not allow binding a reagents, proteins etc.

A "vacuum system" as used herein refers to any system, which allows the provision of vectored vacuum in the analyzer device of the present invention. It may be implemented, for example, by the use of suitable vectored vacuum pumps sucking air or liquid into a specific zone, reservoir or recursive structure element of the device according to the present invention, e.g. into an expansion reservoir or target reservoir, or into a specific direction, leading to a movement of sample or reagent solutions in the device. For instance, a compressed air and vacuum may be used to move membranes which are in contact with the material.

The term "observation window" as used herein relates to an area of the analyzer device which is visible from the outside of the device and is transparent thus allowing the observation of material beneath. The window may, for example, be composed of transparent plastics. In certain embodiments, it may be connected to light sources, e.g. an LED lamp, which illuminated the sector or zone of the device which can be observed. The observation windows, thus, allows the user to (i) determine whether, for example, a color change has occurred during a reaction, (ii) determine the filling state of a recursive structure element or zone, (iii) to determine the status of a reaction or of displacements, e.g. by observing whether, after a rotational movement the filling state has changed.

The term "gas inlet" as used herein refers to an inlet structure at the surface of the analyzer device which allows the introduction of gas, e.g. air, oxygen, nitrogen, CO2 etc. into the device. The inlet may accordingly be connected to an adjacent recursive structure element, or a tubing or tubing system. The tubing system may, in certain embodiments, be connected to one or more recursive structure elements. The inlet may be equipped with a sealing as described herein, e.g. a valve, lid, shutter etc. The sealing and/or the tubing connected to the inlet is preferably air- or gastight.

A "gas outlet" as used herein relates to a similar structure as a gas inlet as define above. It is configured to allow the release or emptying of gas from the device. It may, for example, be connected to an adjacent recursive structure element, or to a tubing or tubing system. It may, in other embodiments, be connected to gas or pressure reservoir zone. It can be equipped with a sealing as described herein e.g. a valve, lid, shutter etc. The sealing and/or the tubing connected to the outlet is preferably air- or gastight.

A "measuring unit" as used herein refers to a module which is capable of implementing a measurement functionality as defined herein, e.g. it may be configured to determine the status, consistency or quality of a sample; to determine the presence or absence of a specific compound, particle, nucleic acid, protein, structure etc. in a sample; to determine the result of a reaction performed on or with the sample; or to determine any parameter connected to the operation, status, performance, handling etc. of the device itself. Preferred examples of measuring units present in the analyzer device of the present invention are a magnetometer or a gyroscope sensor; a hygrometer; a temperature measuring unit; a photometer; pH-meter; an electric conductivity measuring unit; a Raman spectrometer or IR spectrometer; a viscosimeter; a magnetic resonance imaging scanner; an X-ray scanner; a nucleic acid hybrid analyzer and an ultrasonic sensor. Also envisaged is any other functional unit which may be used to perform one or more of the above defined measurement functionalities. For example, the measuring unit may be capable of performing light transmittance measurements, or may be based on turbidimetry, nepholmetry or reflectometry approaches.

In a specific embodiment, the magnetometer or a gyroscope sensor is configured to determine the change in position of the analyzer device. The corresponding information may be processed, e.g. with a microcontroller, in the device and may be provided to the user via a user interface. It may also be compared with pre-defined position information, e.g. according to a rotational movement scheme, e.g. provided in the microcontroller. Alternatively, the information may be transferred to an outside receiving station, e.g. a control apparatus with WLAN or Bluetooth functionality or a mobile phone or similar device. The device, e.g. based on a suitable application or program may inform the use about the operation of the analyzer device and/or provide guidance as to further operational steps to be performed.

In a further embodiment, the hygrometer is configured to determine the humidity inside or outside of the analyzer device. The information obtained upon determination of humidity may, for example, be compared with a predefined set of humidity values, e.g. on a microcontroller or outside of the device on receiving station as mentioned above. Should the measured humidity value in the device be outside of a range of predefined values, suitable countermeasures may be started, e.g. gas/air ventilation or introduction of gas/air, or heating of the device. Should the measured humidity value outside of the device be outside of a range of predefined values, the operation of the device may be stopped and/or the user be accordingly informed.

In a further embodiment, the temperature measuring unit is configured to determine the temperature at various positions in or on the analyzer device. Examples of suitable temperature measuring units are an infrared sensor, a quartz resonator, a platinum measuring resistor or a silicon bandgap temperature sensor. Temperature measuring units may be place at any suitable position of the analyzer. The information obtained upon determination of temperatures may, for example, be compared with a predefined set of temperature values, present e.g. on a microcontroller or outside of the device on a receiving station as mentioned above. Should the measured temperature value in the device be outside of a range of predefined values, suitable countermeasures may be started, e.g. cooling or heating. Should the measured temperature value on or outside of the device, e.g. in the environment, be outside of a range of predefined values, the operation of the device may be stopped and/or the user be accordingly informed.

In a further embodiment, the photometer is configured to detect colors and/or color changes. Examples of suitable photometers include those manufactured by biomed (www.biomed-technics.com). By determining the change of color (i) analytical information on a performed test may be obtained, or (ii) information on the status of tests or reactions. Thus, should there be no color change contrary to expectations, it may be determined that the reaction has not yet finished, or that a malfunction is given. Corresponding information may further be compared with a predefined set of color and/or color plus reaction time values, present e.g. on a microcontroller or outside of the device on a receiving station as mentioned above. Should the measured values in the device be outside of a range of predefined values, suitable countermeasures may be started, e.g. the next rotational movement may be delayed until a color change is detected. Alternatively, the operation of the device may be stopped and/or the user be accordingly informed.

In a further embodiment the pH meter is configured to measure pH values in specific reaction zones or reservoir zones or measurement zones, where the sample or material provided in the recursive structural elements is moved to. Corresponding information may further be compared with a predefined set of pH values, present e.g. on a microcontroller or outside of the device on a receiving station as mentioned above. Should the measured values be outside of a range of predefined values, suitable countermeasures may be started, e.g. neutralizing reagents, acids or bases may be released from reservoir. Alternatively, the operation of the device may be stopped and/or the user be accordingly informed.

In yet another embodiment the Raman spectrometer or IR spectrometer is configured to analyze the composition of a sample or the particle form or size of elements, e.g. cells in a sample. The term "Raman spectrometer" relates to an apparatus capable of registering or producing a Raman spectrum of a substance or of a composition comprising a substance. Typically, Raman spectroscopy uses inelastic or Raman scattering of monochromatic light which interacts with molecular vibrations, phonons or other excitations in a system, resulting in the energy of the light photons being shifted up or down. This shift provides information about vibrational modes in the system. Envisaged examples of Raman spectrometer are resonance Raman spectrometers, surface-enhanced Raman spectrometers, or any other sub-type of Raman spectrometers. The term "IR spectrometer" relates to an apparatus capable of producing an infrared spectrum, which is essentially a graph of infrared light absorbance or transmittance on the vertical axis vs. frequency or wavelength on the horizontal axis. An example of an IR spectrometer envisaged by the present invention is a Fourier transform infrared (FTIR) spectrometer. In a specific embodiment, the IR or the Raman spectrometer may be located on the surface of the device, on which a drop of the liquid can be directly analyzed. Such a functionality allows, inter alia, to detect contaminations in samples or the quality of samples.

In a further embodiment, the viscosimeter is configured to determine the flow conditions, e.g. of a sample, of a reagent or any material within the analyzer device of the present invention, e.g. upon rotational movement or displacement activities. By determining the viscosity (i) analytical information on a performed test may be obtained, or (ii) information on the status of tests or reactions. Thus, should there be a low or high viscosity, e.g. contrary to expectations, it may be determined that a malfunction is given. Corresponding information may further be compared with a predefined set of viscosities, present e.g. on a microcontroller or outside of the device on a receiving station as mentioned above. Should the measured values be outside of a range of predefined values, suitable countermeasures may be started, e.g. the next rotational movement may be delayed until the sample or material displacement to the next recursive structural element or to the next reaction zone etc. is completed. Alternatively, a dilution of the material may be envisaged, e.g. by releasing a diluent, water or a buffer from a reservoir. In a further alternative, the operation of the device may be stopped and/or the user be accordingly informed.

In a further embodiment, the magnetic resonance imaging scanner is configured to determine the structure and/or composition of a sample. Examples of suitable magnetic resonance imaging scanners are ultralow-field magnetic resonance systems with low power requirements and lightweight constructions which use a superconducting quantum interference device (SQUID), acting as a sensitive magnetometer, or pocketable NMR magnets as developed by RWTH Aachen University.

In a further embodiment, the X-ray scanner is configured to determine the structure and/or composition of a sample. Exampies of suitable X-ray scanners are miniature or handheld X-ray scanning systems which typically use backscatter imaging technology.

In a further embodiment, the nucleic acid hybrid analyzer is configured to determine the presence of DNA-DNA or DNA-RNA hybrids within a reaction mixture. For example, the hybrid analyzer may use, for example, signal-amplified, chemiluminescent technology to detect hybridization. Such a technology may, inter alia, involve the employment of a luminometer for amplified chemiluminescent signal detection and result reporting. Further details would be known to the skilled person.

In yet another embodiment, the ultrasonic sensor is configured to determine the volume of the sample or of any material within the analyzer device of the present invention. By determining the volume analytical information on a performed test may be obtained, or (ii) information on the status of tests or reactions. Thus, should there be a low or high volume in a zone or a recursive structural element of the device, e.g. contrary to expectations, it may be determined that a malfunction is given. Corresponding information may further be compared with a predefined set of volume values, present e.g. on a microcontroller or outside of the device on a receiving station as mentioned above. Should the measured values be outside of a range of predefined values, suitable countermeasures may be started, e.g. the next rotational movement may be delayed or accelerated until the sample or material displacement to the next recursive structural element or to the next reaction zone etc. is completed. Alternatively, a volume reduction via the use of reservoirs may be performed. In a further alternative, the operation of the device may be stopped and/or the user be accordingly informed.

The term "microcontroller" as used herein refers to any suitable microcontroller known to the skilled person. The microcontroller may typically comprise one or more of a memory, a microprocessor, an analog-to-digital converter and a transmitter to transmit the data stored in the memory.

The "analog-to-digital converter" as used herein converts measured analogous signals of one or more units used in device to digital signals.

The microprocessor is preferably capable of processing one or more measured data. The memory is preferably capable of storing one or more measured data. A "transmitter" as used in the context of the microcontroller is envisaged to transmit the data stored in the memory. Furthermore, the microcontroller may comprise a time measurement unit which is envisaged to sort one or more of the measured data chronologically. In specific embodiments, during the operation of the analyzer device, the microprocessor may actively record any measured data of one or more measuring units. In a further embodiment, the analyzer device in connection with a time measurement unit is capable of determining when a defined displacement action or rotational move or a defined reaction event took place and under which conditions this occurred. In another embodiment, it may be desirable to store one or more data received from the various measuring units for later analysis, for purposes of user instructions. Thus, a memory device may be provided. In one embodiment, memory may be a non-volatile or persistent memory.

In a further embodiment, the microcontroller may also be configured to transmit data wirelessly to a computer device external of the analyzer device for processing and visualizing data, preferably via a wireless personal area network (WPAN) connectivity such as Bluetooth (RTM) or wireless local area network (WLAN) connectivity. Alternatively, further technologies such as Near Field Communication (NFC) employing electromagnetic induction, ZigBee (RTM), UWB connectivity, or infrared light based connectivity such as Infrared Data Association (IrDA), which would be known to the skilled person and whose specification can be derived from suitable literature sources including IEEE standards for communication, are envisaged.

In another embodiment of the present invention, the microcontroller may further or alternatively control an optical and/or audible warning system. A corresponding optical and/or audible warning system may accordingly also be included in the device. The system may advantageously be programmed for indicating the quality and/or correctness of one or more of the following parameters: temperature, pH, electric conductivity, humidity, position of the analyzer device, sequence of operations, movement or displacement of samples or material, the direction of movement of the device, e.g. which rotational moves are performed, emptying and/or filling status of one or more recursive structure elements, blockage of connections between recursive structure elements or external mechanical impact during operation. Typically, any such parameter obtained, e.g. by using a measuring unit as described herein, may be compared to a predefined value or range of values stored in a memory. Should the parameter value measured be outside of said range, the optical and/or audible warning system may inform the user about this information. Accordingly, measures such as the pausing or stopping of analysis reactions, the delaying of analysis actions or displacement activities, the reduction or raise of temperature, pH, buffer concentrations etc. may be implement ted. Correspondingly obtained data may, as described above, also be transmitted to external unit such as computers or mobile phones, e.g. via wireless personal area network (WPAN) connectivity.

In a further particularly preferred embodiment, the recursive structure elements constitute a diagnostic test system. The system is thus equipped with reagents, units and functionalities which allow a diagnostic analysis of a sample, preferably a whole blood, blood serum, blood plasma, sputum or urine sample. The test system implemented in the device may accordingly comprise reagents and ingredients to perform a diagnostic analysis such as a buffer, a peptide, a protein, an enzyme, a nucleotide, an antibody, or a dye or any other ingredient necessary for the analysis. The analysis may include qualitative and quantitative measurements. The analysis is typically based on a biological or biochemical assay format as known to the skilled person. Such assays typically comprise the following general steps: sample processing and manipulation, target specific discrimination or identification, signal or target amplification, signal detection and/or interpretation, and signal enhancement and noise filtering. The assays may include end point assays, i.e. assays in which a single measurement is performed after a fixed incubation period, or kinetic assays, i.e. assays in which measurements are performed multiple times over a fixed time interval. The assays may, for example, include assays in the biological activity of a sample is tested, ligand binding assay, where a ligand binds a receptor, or immunoassays, where the response is an antigen antibody binding type reaction. Also envisaged are assays based on nucleic acids, involving for examples hybrid formation between DNA and DNA molecules, or DNA and RNA molecules, or between artificial nucleic acids and DNA or RNA molecules etc. Further envisaged are enzyme assays, where, for example, enzymes may be tested via their highly repeating activity on a large number of substrates when loss of a substrate or the making of a product may have a measurable attribute like color or absorbance at a particular wavelength or light or electrochemiluminescence or electrical/redox activity. Also contemplated are assays based on radioisotopes, where radiolabeled substrates may be used in radioimmunoassays or equilibrium dialysis assays. Further envisaged assays may be based on the use of polymerase chain reactions, where a DNA or RNA template is amplified and/or detected. Also possible are combinations of any of the above mentioned assays.

The analysis may, for example, be based on the detection of proteins or protein fragments via binding partners such as antibodies, lectins, peptides or non-immunoglobulin protein comprising an ankyrin-repeat protein (DARPin), affibody molecule, adnectin, anticalin, affilin, avimer, knottin, fynomer, phylomer, artificial antibody mimic and a kunitz domain peptide. The analysis may, in further embodiment, be based on chemiluminescent signal detection, e.g. based on luminol compounds which can be detected after cupric ions are dissolved from hybrids. Further analysis may include the use of magnetic particles, e.g. comprising one or more of the above mentioned entities, e.g. antibodies or other interactors, which can be bound, selected and/or moved over magnetic zones within the analyzer device or by the provision of temporary magnetic field. Also envisaged are analysis techniques based on fluorescence in situ hybridization or ELISA techniques. Furthermore, analysis based on microarray structures, which may, for example be provided in one or more specific reaction zones of the device, are envisaged. Reagents, ingredients, buffers etc. useful for the performance of an assays as mentioned above may, for example, be provided to the device during the manufacturing process, i.e. 3D printing, e.g. by filling in the reagents. Furthermore, in specific embodiments, such reagents, e.g. antibodies, nucleic acids, enzymes, nanoparticles etc. may be immobilized at certain zones of the device. Such functionalized areas may be introduced into the device during the 3D-printing as described above, e.g. during an interruption of the printing procedure. In further, preferred embodiments, the ingredients, reagents etc. may be provided in the form of a reagent pack or cartridge, e.g. as described herein above, which may be introduced into the device via a chute structure as defined herein. Accordingly, zones of immobilized elements may be provided via said cartridge. The cartridge may further allow the presence of different types of ingredients, e.g. liquid ingredients, solid ingredients or gaseous ingredients, which all may be release upon the correct placing of the cartridge in the device.

In addition to suitable reagents and ingredients necessary to perform any of the above tests, the analyzer device may comprise suitable measuring units to detect corresponding biological or biochemical reaction results. In simple, preferred embodiment, the measuring unit is a photometer configured to detect colors and/or color changes.

In a particularly preferred embodiment the test system is a test system based on the CRISPR/Cas method. The "CRISPR/Cas method" is a biochemical method to specifically cut and modify DNA and also known as genome editing. Genes in a genome can generally be inserted, removed or switched off with the CRISPR/Cas system, nucleotides in a gene can also be changed. The effect of the suppression of a gene by CRISPR/Cas has various similarities to that of RNA interference, since short RNA fragments of about 18 to 20 nucleotides mediate the binding to the target in both bacterial defense mechanisms. Cas proteins can typically bind certain RNA sequences as ribonucleoproteins. The Cas endonuclease (e.g. Cas9, Cas5, Csn1 or Csx12) can bind to certain RNA sequence (e.g. crRNA repeat) and cut DNA in the immediate vicinity. This crRNA repeat sequence forms a secondary RNA structure and is then bound by Cas which alters its protein folding allowing the target DNA to be bound by the RNA. Furthermore, the presence of a PAM motif (protospacer adjacent motif) in the target DNA is necessary to activate Cas. The DNA is cut three nucleotides before PAM. The crRNA repeat sequence is followed by a sequence binding to the target DNA (crRNA spacer); both sequences are together called crRNA. The second part of the crRNA is the crRNA-spacer sequence in the function of a variable adapter, which is complementary to the target DNA and binds to the target DNA. An additional RNA (tracrRNA, or trans-acting CRISPR RNA) is also required. tracrRNA is partially complementary to crRNA, so that they bind to each other. tracrRNA binds to a precursor crRNA, forms an RNA double helix and is converted into the active form by RNase III. These properties bind the DNA and cut it from the endonuclease function near the binding site. The DNA repair of the generated double strand break is carried out by homology-directed repair (HDR) or non-homologous end joining (NHEJ).

To become applicable in the diagnostics field, the CRISPR/Cas method may be adapted to the specific target and its read-out principle be modified. In a preferred embodiment, a CRISPR/Cas-based diagnosis may be performed with specific Cas enzymes which, instead of DNA, bind to RNA molecules. For example, an RNA-guided RNAse such as Cas13a (aka C2c2) or any similar activity may be used. Upon recognition of its RNA target, e.g. a viral or bacterial sequence, the Cas13a, which can be activated via specific CRSIPR RNAs (crRNAs), may engage in collateral cleavage of nearby non-targeted RNA sequences, e.g. reporter sequences. The corresponding crRNA-programmed collateral cleavage activity thus allows Cas13a to detect the present of specific RNA by nonspecific degradation of labeled RNA. One possibility of using this principle is to employ a RNA reporter substrate which comprises a dye and a quencher element, e.g. a fluorescent reporter molecule on one end and a quencher on the other. In the absence of an RNases activity, which is provided in the present scenario by the RNA-guided Cas13a RNAse, the physical proximity of the quencher dampens fluorescence from the fluorescent reporter to extremely low levels. When the Cas13a is activated by binding to its target sequence, however, the RNA substrate is cleaved, and the fluorescent reporter and quencher are spatially separated in solution. This causes the fluorescent reporter to emit a signal when excited by light of an appropriate wavelength. Fluorescence can subsequently detected with a measuring unit such as a filter-based or monochromator-based fluorometer. In further embodiments, the use of Cas enzymes, which bind to double stranded nucleic acids, e.g. DNA, is envisaged.

The approach may advantageously further be combined with suitable amplification steps for the target nucleic acid. For example, the amplification of target nucleic acids may be performed with the help of a Recombinase Polymerase Amplification (RPA) procedure. This procedure is an isothermal DNA amplification technique consisting of three essential enzymes: a recombinase, single-stranded DNA-binding proteins (SSBs), and a strand displacing polymerase. RPA typically overcomes technical difficulties present in other amplification strategies such as polymerase chain reaction (PCR), by not requiring temperature regulation as the enzymes all operate at a constant temperature of about 37°C. RPA accordingly does not require any temperature cycling for global melting of a double-stranded template and primer annealing with an enzymatic approach based on in vivo DNA replication and repair. Typically, recombinase-primer complexes scan double-stranded DNA and facilitate strand exchange at complementary sites. The strand exchange is subsequently stabilized by SSBs, allowing the primer to stay bound. Spontaneous disassembly of the recombinase may occur in an ADP-bound state, allowing a strand-displacing polymerase to invade and extend the primer, allowing amplification without complex instrumentation. The procedure advantageously allows cyclic repetition in a temperate range of about 37-42°C and typically results in exponential DNA amplification. It is preferred to use the Bacillus subtilis Pol I (Bsu) enzyme as the strand-displacing polymerase, T4 uvsX as the recombinase, and T4 gp32 as the single-stranded DNA binding protein. Also envisaged is the use of a corresponding kit provided by the Company TwistDx, or any other similar kit. Further information may, for example, be derived from suitable literature sources such as Daher et al., 2016, Clinical Chemistry, 62, 7, 947-958.

For RNA amplification a reverse transcription step may be added. Subsequent to the RPA procedure any produced DNA fragment may be converted into a RNA molecule by T7 transcription, which is then followed by the Cas13a detection as described above.

In case a DNA detection is performed, the above described reporter activation scheme may be followed, e.g. by using a double stranded nucleic acid reporter molecule comprising a dye and a quencher. In such a scenario the use of transcriptases after an amplification step may be skipped. Also envisaged are hybrid application forms combining both ds and ss nucleic acid forms, or the use of Cas enzymes detecting both DNA and RNA molecules.

In a preferred embodiment, the diagnostic test system based on the CRISPR/Cas method comprises the following elements:
(i) An optional, first recursive structure element or reaction zone within a recursive structure element which comprises a reverse transcriptase activity, a suitable buffer and nucleotides in a suitable concentration; this first recursive structure element may advantageously be used in case RNA molecules shall be detected. In case a DNA molecule is to be detected, the system may not comprise said first recursive structure element but only the subsequently mentioned entities.
(ii) A second, preferably adjacent recursive structure element or reaction zone within a recursive structure element (alternatively first recursive structure element or reaction zone within a recursive structure element if only a DNA molecule is to be detected) which comprises a recombinase, single-stranded DNA-binding proteins (SSBs), and a strand displacing polymerase, preferably the Bacillus subtilis Pol I (Bsu) enzyme as the strand-displacing polymerase, T4 uvsX as the recombinase, and T4 gp32 as the single-stranded DNA binding protein, as well as a suitable buffer for RPA and nucleotides in a suitable concentration.
(iii) A third, preferably adjacent recursive structure element or reaction zone within a recursive structure element (alternatively second recursive structure element or reaction zone within a recursive structure element if only a DNA molecule is to be detected) which comprises a transcriptase, preferably a T7 transcriptase, as well as a suitable buffer and nucleotides in a suitable concentration.
(iii) A fourth, preferably adjacent recursive structure element or reaction zone within a recursive structure element (alternatively third recursive structure element or reaction zone within a recursive structure element if only a DNA molecule is to be detected) which comprises an activated RNA binding enzyme of the Cas protein family, preferably a Cas13a enzyme, one or more RNA reporters, preferably an RNA reporter substrate which comprises a dye and a quencher element and allows for the emittance of fluorescence upon cleavage of the RNA reporter molecule, as well as a suitable buffer.

Optionally included may further be a measuring unit, preferably a photometer, more preferably a fluorometer such as a filter-based or monochromator-based fluorometer.

In further embodiments, a heating unit or heating zone may optionally be included, wherein said heating unit is configured to have a connection with one, more or all other recursive structures.

The present invention further envisages alternative approaches for the detection of target nucleic acids. Such alternative approaches may, for example, include the employment of different enzymes, the production of hybrid nucleic acid structures and/or the use of a hybrid analyzer as described herein above.

In another aspect the present invention relates to a system comprising the analyzer device as defined herein above and at least one reagent pack. The reagent pack may be provided, as described herein, as cartridge. The cartridge may accordingly, in specific embodiments comprise a buffer, an acid, a base, a peptide, a protein, an enzyme, an antibody, and/or a dye. Also envisaged is any other suitable ingredient for the operation of the device. Also envisaged is the presence of a measuring unit or other functional units in the cartridge. The cartridge may, according to further embodiments, be provided at a different location than the device, and delivered to the place where the device is used. Alternatively, the system may be provided as described. The present invention accordingly contemplates the provision of the system in any chronology, chronological order or sequence.

In another aspect the present invention relates to a method for in vitro diagnosis of a viral or bacterial infection or of cancer. The method advantageously comprises the diagnostic analysis of a sample in an analyzing device as defined herein or a system as defined herein. The method may, in specific embodiments, comprise the performance of an assay as defined herein above. It is preferred that the method is a diagnostic CRISPR/Cas method. It is particularly preferred that the method comprises the following steps:
(i) Optional reverse transcription, e.g. with a reverse transcriptase activity, of a target RNA, e.g. derived from a sample, to yield a DNA molecule. This step may only be implemented in case the target is an RNA molecule.
(ii) Recombinase Polymerase Amplification (RPA) of a DNA molecule, e.g. as provided in step (i) or as provided in a sample comprising the use of a recombinase, single-stranded DNA-binding proteins (SSBs), and a strand displacing polymerase, preferably of the Bacillus subtilis Pol I (Bsu) enzyme as the strand-displacing polymerase, T4 uvsX as the recombinase, and T4 gp32 as the single-stranded DNA binding protein.
(iii) Transcribing the obtained DNA molecules of step (ii) into RNA molecules with a transcriptase, preferably a T7 transcriptase.
(iv) Detecting the presence of a target molecule by contacting the RNA molecules obtained in step (iii) with an activated RNA binding enzyme of the Cas protein family, preferably a Cas13a enzyme in the presence of one or more RNA reporters, preferably an RNA reporter substrate which comprises a dye and a quencher element. This allows for the emittance of fluorescence upon cleavage of the RNA reporter molecule, which is indicative of the presence of a target RNA molecule.
(v) Optionally detecting and/or quantifying the emitted fluorescence with a fluorometer, preferably a filter-based or monochromator-based fluorometer.

A method for in vitro diagnosis of a viral or bacterial infection or of cancer as described above may further comprise one or more of the steps of
(i) purifying the sample before analysis;
(ii) performing a reaction with the sample in one or more recursive structure element as defined herein;
(iii) detecting reaction results preferably by observation in a window or via a measuring unit.

It is preferred that these additional steps are implemented within the method as described above, e.g. combined with the following method steps (i) optional reverse transcription of an RNA target molecule, e.g. derived from a sample as, (ii) Recombinase Polymerase Amplification (RPA) of a DNA target molecule, e.g. as provided in step (i) or as provided in a sample, comprising the use of a recombinase, single-stranded DNA-binding proteins (SSBs), and a strand displacing polymerase, (iii) transcribing the obtained DNA molecules of step (ii) into RNA molecules with a transcriptase, preferably a T7 transcriptase, (iv) detecting the presence of a target molecule by contacting the RNA molecules obtained in step (iii) with an activated RNA binding enzyme of the Cas protein family, preferably a Cas13a enzyme in the presence of one or more RNA reporters, preferably an RNA reporter substrate which comprises a dye and a quencher element. Samples to be used may accordingly be pretreated, e.g. by the additional of RNAse inhibitors, preferably RNAse inhibitors which do not affect the Cas proteins used, the addition of coagulation inhibitors etc. The detection may accordingly be performed with the help of a fluorometer as described above, or by direct observation via an observation window.

In another aspect the present invention relates to the use of the analyzing device as defined herein or the system as defined herein for the diagnostic analysis of a sample. The sample is preferably a sample as defined herein, e.g. a whole blood, blood serum, blood plasma, sputum or urine sample. The use may include the performance of assay steps or assay procedures as mentioned herein above. It is preferred that the diagnostic analysis is based on a CRSPR/Cas diagnostic approach as described herein above. Also envisaged are further suitable diagnostic approaches known to the skilled person. The diagnostic approach may include the detection of affection by a disease or infection, the detection of biological elements such as nucleic acids or proteins in a sample, e.g. derived from microbes or viruses, or any other organism in a sample independent of a disease or infection, e.g. when analyzing environmental probes, or consortia of bacterial or organisms etc.

It is further preferred to use the analyzing device as defined herein or the system as defined herein for the diagnostic analysis of a viral or bacterial infection, or for the diagnosis of cancer. A viral infection which may advantageously be diagnosed is an infection by Zika virus or Dengue virus. The present invention further envisages the diagnosis of novel or emerging diseases, e.g. transmitted by viruses or bacteria, or of diseases of epidemic or pandemic dimension, e.g. transmitted by virus or bacteria. Also envisaged is the diagnosis of an infection by a eukaryotic organism, e.g. a protozoon such as Plasmodium.

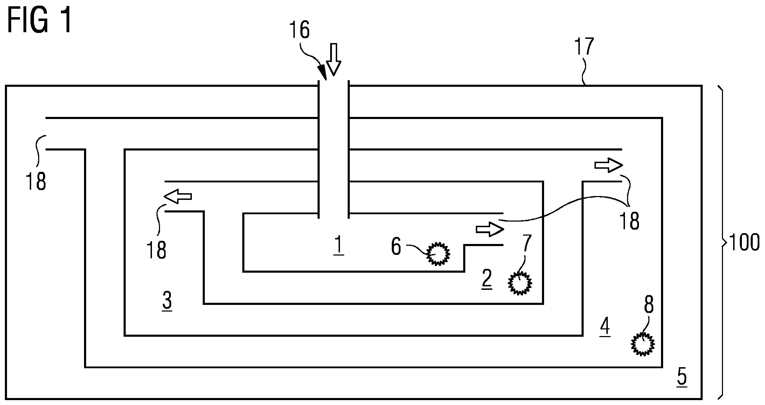

Turning now to FIGURE 1, an analyzer device 100 is shown. The device comprises a sleeve 17. The device comprises an entry opening 16 which is connected to recursive structure element 1. The recursive structure element 1 is connected via connection 18 to recursive structure element 2. The device additionally comprises further recursive structure elements 3, and 4, and 5, which are respectively connected via connections 18. The recursive structure elements comprise reagents 6, 7 and 8, e.g. for the performance of diagnostic assays. The entrance and displacement of the sample or material in the recursive structure elements is shown with arrows.

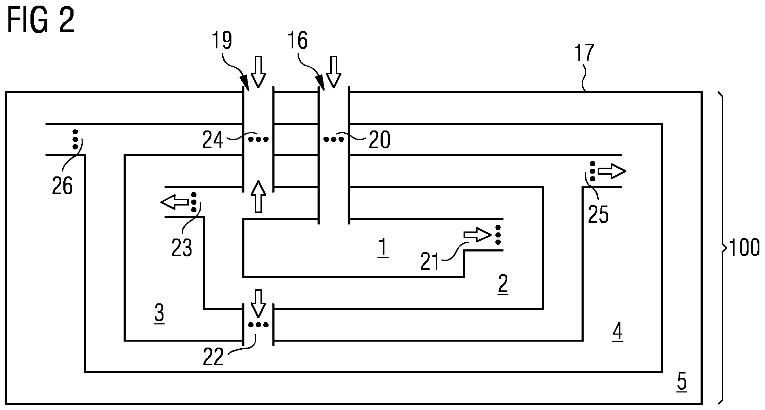

FIGURE 2 illustrates a further, alternative analyzer device 100. The device comprises a sleeve 17. The device comprises an entry opening 16 and an alternative entry opening 19 which are connected to recursive structure element 1 or 2, respectively. The recursive structure element 1 and 2 are connected by closable connection 21. The device additionally comprises further recursive structure elements 3, 4, and 5, which are connected to recursive structure elements 1 and 2 by closable connections 22, 23, 25 and 26. The entry opening 16 and the alternative entry opening 19 are also equipped with closable connections 20 and 24. The displacement of the sample or material in the recursive structure elements is shown with arrows.

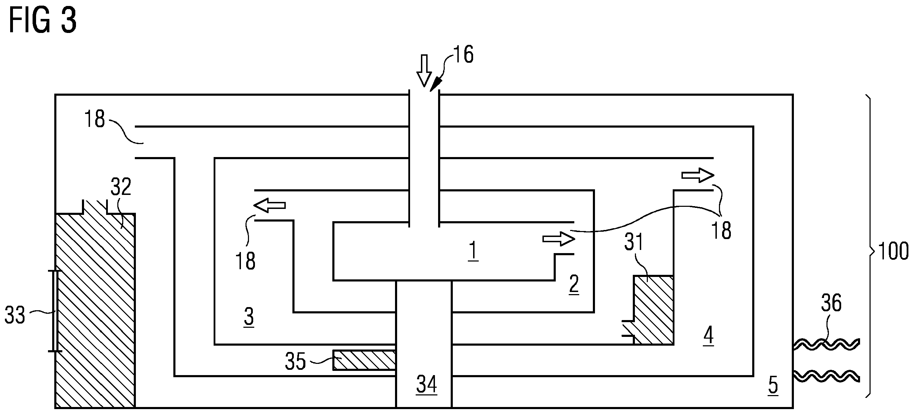

FIGURE 3 illustrates an analyzer device 100 which comprises recursive structure elements 1, 2, 3, 4 and 5 which are connected by connections 18. The device further comprises an entry opening 16 which connects with recursive structure elements 1. The device further comprises a reservoir zone 31, a measuring zone 32, an observation window 33, a heatable reaction zone 34, a second reservoir zone 35 and a gas inlet/outlet 36. The displacement of the sample or material in the recursive structure elements is shown with an arrow.

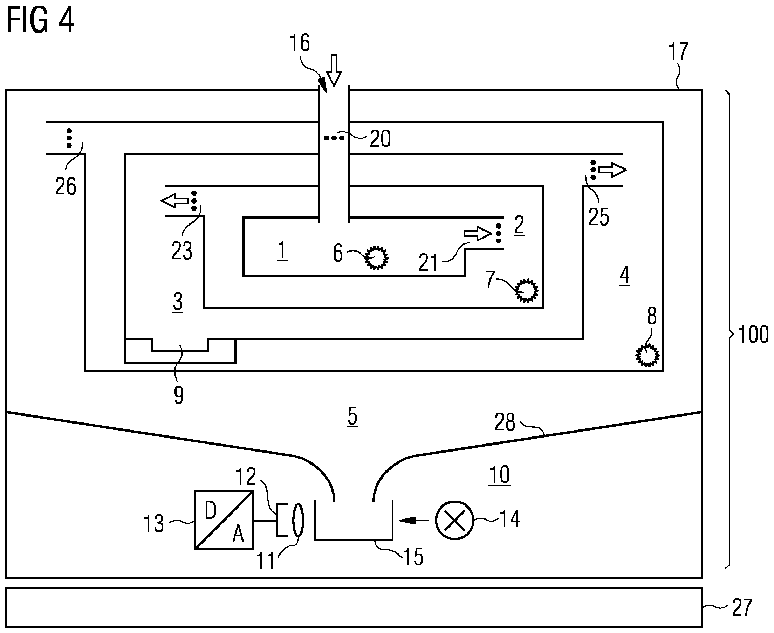

FIGURE 4 shows an analyzer device 100 which comprises recursive structure elements 1, 2, 3, 4 and 5. The device comprises sleeve 17. The recursive structure elements are connected with closable connections 21, 23, 25 and 26. The device further comprises an entry opening 16 which is equipped with closable connection 20. The recursive structure elements comprise reagents 6, 7 and 8 for the performance of diagnostic assays. The device is further equipped with a specific reaction zone for RPA 9. Probes which have passed recursive structure elements 1, 2, 3, 4 and 5 pass funnel 28, and arrive at receptacle 15 which are provided in measuring chamber 10. The measurement is performed with light source 14, lens 11, CCD sensor 12, and analog digital converter 13. The device is placed on external magnetic field inducer 27.

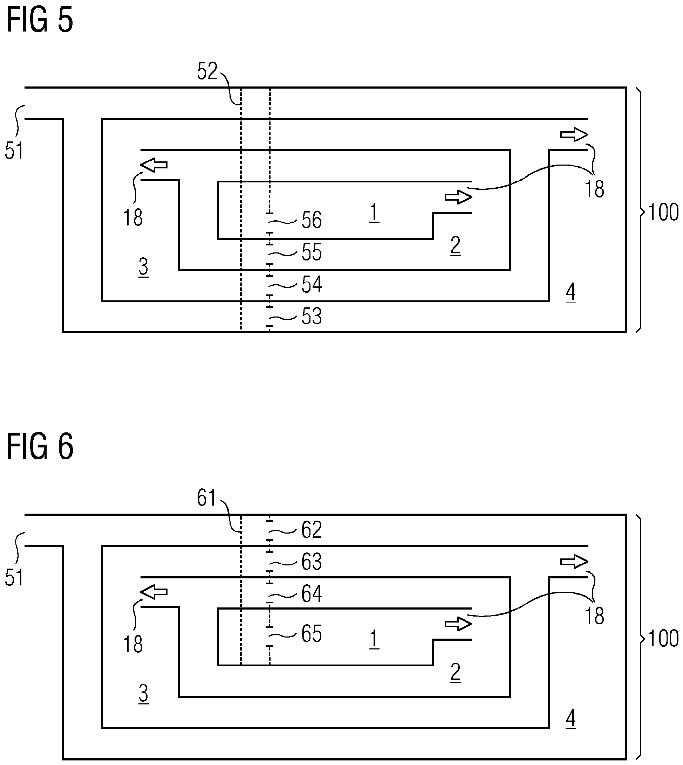

FIGURE 5 shows an analyzer device 100 according to the present invention. The analyzer device comprises an outlet opening 51, a chute 52, which connects recursive structure elements 1, 2, 3, and 4. The analyzer further comprises connections 18 between said recursive structure elements. Within the chute 52 interaction zones 53 to 56 are provided for the release of reagents or samples introduced via a cartridge or sample provider into the analyzer device.

FIGURE 6 shows a further embodiment of an analyzer device 100, comprising an outlet opening 51 and chute 61, which reaches recursive structure element 1, providing interaction zones 62 to 65 for the release of reagents or samples introduced via a cartridge or sample provider into the analyzer device in recursive structure elements 1, 2, 3, and 4. The analyzer further comprises connections 18 between said recursive structure elements.

FIGURE 7 shows an analyzer device 100 in a rolling device 101. The analyzer device comprises an attachment point of rotation on the left hand side 71 and an attachment point of rotation on the right hand side 72. The axis of rotation 73 may be inclined at different angles.

FIG. 8 shows a vacuum chamber 81 comprising analyzer device 100. The analyzer device may comprise an opening 82, which can be opened/closed to allow air or gases to exit. Connected to outlet 83 is a vacuum pump 84.

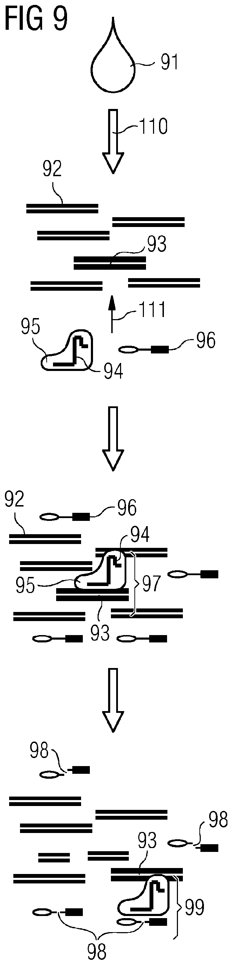

Fig. 9 illustrates a diagnostic approach for the detection of specific nucleic acids. Starting with a liquid sample 91, double stranded nucleic acids 92 and 93 are generated 110. Nucleic acid 93 is a target nucleic acid comprising a mutation or single nucleotide polymorphism. The generation of double stranded nucleic acids 92 and 93 may be started with a single stranded nucleic acid, or a double stranded nucleic acid and involve an amplification step 110. Cas enzyme 95, guide RNA 94 and reporter molecules 96 are subsequently added 111. A complex 97 of Cas enzyme 95 and guide RNA 94 binds to target nucleic acid 93. After having bound to its specific target 93 the Cas enzyme 95 and guide RNA 94 nonspecifically binds to reporter molecule 96 to form complex 99. Thereby the reporter molecule is cleft into two portions 98, one of which emits a detectable signal.

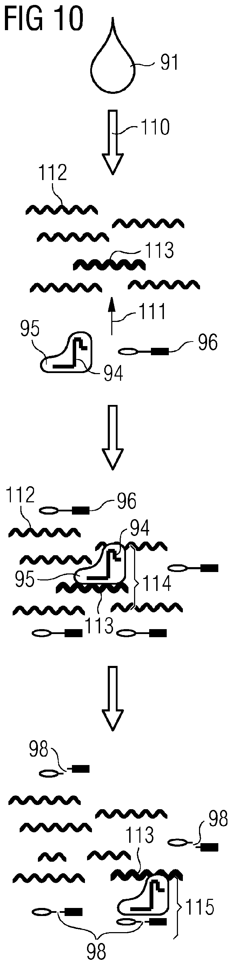

Fig. 10 illustrates a diagnostic approach for the detection of specific nucleic acids. Starting with a liquid sample 91, single stranded nucleic acids 112 and 113 are generated 110. Nucleic acid 113 is a target nucleic acid comprising a mutation or single nucleotide polymorphism. The generation of single stranded nucleic acids 112 and 113 may be started with a single stranded nucleic acid and involve an amplification step 110. Cas enzyme 95, guide RNA 94 and reporter molecules 96 are subsequently added 111. A complex 114 of Cas enzyme 95 and guide RNA 97 binds to target nucleic acid 113. After having bound to its specific target 113 the Cas enzyme 95 and guide RNA 94 nonspecifically binds to reporter molecule 96 to form complex 115. Thereby the reporter molecule is cleft into two portions 98, one of which emits a detectable signal.

## Claims

1. An analyzer device for analyzing a sample comprising:
(i) at least two nested recursive structure elements;
(ii) wherein each of the recursive structure elements has at least one connection to at least one of the other recursive structure elements; and
(iii) at least one measurement functionality;
wherein said analyzer device is configured to allow, via said connections, a displacement of the sample and of material present in a recursive structure element, such as liquid material, to the next recursive structure element by gravity in the form of a rotation of the analyzer device in a 3D-coordinate system or, optionally, with additional forces such as capillary motion or air or liquid pressure, wherein at least one of the recursive structure elements comprises at least one opening at the surface, configured to receive and/or release the sample for analyzing, wherein the recursive structure elements are suitable to hold liquids within, wherein all recursive structure elements are part of a fluid communication system providing direct or indirect connections between said recursive structure elements, wherein the analyzer device comprises within at least one recursive structure element or in separate recursive structure elements a measuring zone implementing the measurement functionality; and wherein said connections have the form of straight or curved elongations with two openings.

2. The analyzer device according to claim 1, wherein the, preferably all, recursive structure elements are connected by at least one chute comprising an opening at the surface of the device, configured to receive a cartridge comprising one or more reagents in the form of a reagent pack, and/or one or more samples.

3. The analyzer device according to claim 2, wherein said opening is sealable, preferably by a valve, a shutter, a lid, a plug or an adhesive tape.

4. The analyzer device according to any of the preceding claims, wherein said recursive structure element comprises, essentially consists of or consists of material, which is printable by a 3D-printer, preferably a plastic or metal material.

5. The analyzer device according to claim 4, wherein said plastic material is a thermoplastic, a thermosetting plastic or a light-activated resin.

6. The analyzer device according to any of the preceding claims, wherein said direct or indirect connections between said recursive structure elements are closable in a controlled manner.

7. The analyzer device according to any of the preceding claims, wherein said sample for analyzing is an inorganic or organic liquid sample, preferably a body fluid sample such as whole blood, blood serum, blood plasma, sputum or urine.

8. The analyzer device according to any of the preceding claims, wherein the form of the analyzer device is a three- to ten-sided prism, a cylinder or a cuboid.

9. The analyzer device according to any of the preceding claims, wherein the analyzer device comprises within at least one recursive structure element or in separate recursive structure elements a reaction zone, or a reservoir zone or combinations thereof.

10. The analyzer device according to any of the preceding claims, wherein the analyzer device additionally comprises within at least one recursive structure element or in separate recursive structure elements, or in connection with at least one recursive structure element: a measuring unit implementing the measurement functionality, an energy source such as a battery or an accumulator, a heating unit, a cooling unit, a timer unit, a sterilization unit, a magnetic stirrer, a vacuum system, an observation window, a gas inlet, and/or a gas outlets.

11. The analyzer device according to claim 10, wherein said measuring unit is a magnetometer or a gyroscope sensor configured to determine the change in position of the analyzer device; a hygrometer configured to determine the humidity inside or outside of the analyzer device; a temperature measuring unit configured to determine the temperature at various positions in or on the analyzer device, preferably an infrared sensor, a quartz resonator or a platinum measuring resistor or a silicon bandgap temperature sensor; a photometer configured to detect colors and/or color changes; pH-meter; an electric conductivity measuring unit; a Raman spectrometer or IR spectrometer configured to analyze the composition or particle size of the sample; a viscosimeter; a magnetic resonance imaging scanner; an X-ray scanner; a nucleic acid hybrid analyzer or an ultrasonic sensor configured to determine the volume of the sample.

12. The analyzer device according to any one of the preceding claims, wherein said recursive structure elements constitute a diagnostic test system, which comprises reagents to perform a diagnostic analysis such as a buffer, a peptide, a protein, an enzyme, a nucleotide, an antibody, a dye, and a measuring unit to detect biochemical reaction results, preferably a photometer configured to detect colors and/or color changes.

13. The analyzer device according to claim 12, wherein said test system is a CRISPR/Cas test system, comprising at least: (i) an optional first recursive structure element or reaction zone within a recursive structure element which comprises a reverse transcriptase activity, a buffer and nucleotides; (ii) a second, preferably adjacent, recursive structure element or reaction zone within a recursive structure element which comprises a recombinase, single-stranded DNA-binding proteins (SSBs), and a strand displacing polymerase, a buffer for RPA and nucleotides; (iii) a third, preferably adjacent recursive structure element or reaction zone within a recursive structure element which comprises a transcriptase, preferably a T7 transcriptase, a suitable buffer and nucleotides; and (iv) a fourth, preferably adjacent, recursive structure element or reaction zone within a recursive structure element which comprises an activated RNA binding enzyme of the Cas protein family, preferably a Cas13a enzyme, one or more RNA reporters, preferably an RNA reporter which comprises a dye and a quencher element, and a buffer; and optionally a measuring unit, preferably a fluorometer.

14. The analyzer device according to any of the preceding claims, wherein one or more of the recursive structure elements comprises a microcontroller.

15. The analyzer device according to claim 14, wherein said microcontroller controls an optical and/or audible warning system programmed for indicating the quality and/or correctness of one or more of the following parameters: temperature, pH, electric conductivity, humidity, position of the analyzer device, sequence of operations, movement of samples, direction of movement of the device, emptying and/or filling status of a recursive structure element, blockage of connections between recursive structure elements or external mechanical impact during operation.

16. The analyzer device according to any of the preceding claims, wherein said analyzer device rotates manually or automatically, preferably via a rotating element connected on at least one side of the analyzer device, or by a roller element, in a 3D-coordinate system.

17. A method for producing an analyzer device as defined in any of the preceding claims, wherein said method comprises 3D-printing of the nested recursive structure elements of the analyzer device and the direct or indirect connections between said recursive structure elements, wherein the recursive structure elements are suitable to hold liquids within, wherein all recursive structure elements are part of a fluid communication system providing direct or indirect connections between said recursive structure elements, wherein the printing process is interrupted at least once to introduce a prefigured measuring unit and optionally one or more prefigured parts of the analyzer device such as a microcontroller, a reagent, a peptide, a protein, an enzyme, an antibody, a composition, an energy source, a heating unit, a cooling unit, a timer unit, a sterilization unit, and/or a magnetic stirrer; wherein said connections have the form of straight or curved elongations with two openings.

18. A system comprising the analyzer device as defined in any one of claims 1 to 16 and at least one reagent pack, wherein the reagent pack is provided as cartridge, preferably comprising a buffer, an acid, a base, a peptide, a protein, an enzyme, an antibody, and/or a dye.

19. A method for in vitro diagnosis of a viral or bacterial infection or of cancer comprising the diagnostic analysis of a sample in an analyzing device according to any one of claims 1 to 16 or the system according to claim 18.

20. The method according to claim 19 comprising one or more of the steps of
(i) purifying the sample before analysis;
(ii) performing a reaction with the sample in one or more recursive structure element as defined in any one of claims 1 to 17;
(iii) detecting reaction results preferably by observation in a window or via a measuring unit.

21. Use of the analyzing device according to any one of claims 1 to 16 or the system according to claim 18 for the diagnostic analysis of a sample.

22. The use of claim 21, wherein said diagnostic analysis is the CRISPR/Cas-based diagnosis of a viral or bacterial infection or of cancer.

## Patentansprüche

1. Analysatorvorrichtung zum Analysieren einer Probe, Folgendes umfassend:
(i) mindestens zwei verschachtelte rekursive Strukturelemente;
(ii) wobei jedes der rekursiven Strukturelemente mindestens eine Verbindung mit mindestens einem der anderen rekursiven Strukturelemente aufweist; und
(iii) mindestens eine Messfunktionalität;
wobei die Analysatorvorrichtung dazu ausgelegt ist, über die Verbindungen eine Verdrängung der Probe und des Materials, das in einem rekursiven Strukturelement vorhanden ist, wie beispielsweise flüssiges Material, an das nächste rekursive Strukturelement durch Gravitation in der Gestalt einer Drehbewegung der Analysatorvorrichtung in einem 3D-Koordinatensystem oder gegebenenfalls mit zusätzlichen Kräften zu ermöglichen, wie beispielsweise Kapillarbewegung oder Luft- oder Flüssigkeitsdruck, wobei mindestens eines der rekursiven Strukturelemente mindestens eine Öffnung an der Oberfläche umfasst, die dazu ausgelegt ist, die Probe zum Analysieren aufzunehmen und/oder freizusetzen, wobei die rekursiven Strukturelemente geeignet sind, um Flüssigkeiten im Inneren zu halten, wobei alle rekursiven Strukturelemente Teil eines Fluidverbindungssystems sind, das unmittelbare oder indirekte Verbindungen zwischen den rekursiven Strukturelementen bereitstellt, wobei die Analysatorvorrichtung in mindestens einem rekursiven Strukturelement oder in separaten rekursiven Strukturelementen eine Messzone umfasst, welche die Messfunktionalität implementiert;
und wobei die Verbindungen die Gestalt von geraden oder gekrümmten Verlängerungen mit zwei Öffnungen aufweisen.

2. Analysatorvorrichtung nach Anspruch 1, wobei die, vorzugsweise alle, rekursiven Strukturelemente durch mindestens eine Rinne verbunden sind, die eine Öffnung an der Oberfläche der Vorrichtung umfasst, die dazu ausgelegt ist, eine Patrone aufzunehmen, die ein oder mehrere Reagenzien in der Gestalt eines Reagenzpakets und/oder eine oder mehrere Proben umfasst.

3. Analysatorvorrichtung nach Anspruch 2, wobei die Öffnung versiegelbar ist, vorzugsweise durch ein Ventil, eine Klappe, einen Deckel, einen Pfropfen oder ein Klebeband.

4. Analysatorvorrichtung nach einem der vorhergehenden Ansprüche, wobei das rekursive Strukturelement vorzugsweise ein Kunststoffmaterial oder metallisches Material umfasst, im Wesentlichen daraus besteht oder aus Material besteht, das durch einen 3D-Drucker druckbar ist.

5. Analysatorvorrichtung nach Anspruch 4, wobei das Kunststoffmaterial ein Thermoplast, ein wärmeaushärtender Kunststoff oder ein Licht-aktiviertes Harz ist.

6. Analysatorvorrichtung nach einem der vorhergehenden Ansprüche, wobei die unmittelbaren oder indirekten Verbindungen zwischen den rekursiven Strukturelementen in einer gesteuerten Weise schließbar sind.

7. Analysatorvorrichtung nach einem der vorhergehenden Ansprüche, wobei die Probe zum Analysieren eine anorganische oder organische Flüssigkeitsprobe, vorzugsweise eine Körperflüssigkeitsprobe, wie beispielsweise Vollblut, Blutserum, Blutplasma, Sputum oder Urin, ist.

8. Analysatorvorrichtung nach einem der vorhergehenden Ansprüche, wobei die Gestalt der Analysatorvorrichtung ein dreibis zehnseitiges Prisma, ein Zylinder oder ein Kuboid ist.

9. Analysatorvorrichtung nach einem der vorhergehenden Ansprüche, wobei die Analysatorvorrichtung in mindestens einem rekursiven Strukturelement oder in separaten rekursiven Strukturelementen eine Reaktionszone oder eine Reservoirzone oder Kombinationen davon umfasst.

10. Analysatorvorrichtung nach einem der vorhergehenden Ansprüche, wobei die Analysatorvorrichtung zusätzlich in mindestens einem rekursiven Strukturelement oder in separaten rekursiven Strukturelementen oder im Zusammenhang mit mindestens einem rekursiven Strukturelement Folgendes umfasst: eine Messeinheit, welche die Messfunktionalität implementiert, eine Energiequelle, wie beispielsweise eine Batterie oder einen Akkumulator, eine Heizeinheit, eine Kühleinheit, eine Zeitgebereinheit, eine Sterilisationseinheit, ein Magnetrührwerk, ein Vakuumsystem, ein Beobachtungsfenster, einen Gaseinlass und/oder Gasauslässe.

11. Analysatorvorrichtung nach Anspruch 10, wobei die Messeinheit ein Magnetometer oder ein Gyroskopsensor ist, das dazu ausgelegt ist, die Positionsveränderung der Analysatorvorrichtung zu bestimmen; ein Hygrometer ist, das dazu ausgelegt ist, die Luftfeuchtigkeit innerhalb oder außerhalb der Analysatorvorrichtung zu bestimmen; eine Temperaturmesseinheit ist, die dazu ausgelegt ist, die Temperatur an verschiedenen Positionen in oder auf der Analysatorvorrichtung zu bestimmen, vorzugsweise ein Infrarotsensor, ein Quarzresonator oder ein Platin-Messwiderstand oder ein Silizium-Bandlückentemperatursensor; ein Photometer ist, das dazu ausgelegt ist, Farben und/oder Farbveränderungen zu detektieren; ein pH-Messinstrument ist; eine elektrische Leitfähigkeitsmesseinheit ist; ein Raman-Spektrometer oder IR-Spektrometer ist, das dazu ausgelegt ist, die Zusammensetzung oder Teilchengröße der Probe zu analysieren; ein Viskosimeter ist; ein Magnetresonanzbildgebungsscanner ist; ein Röntgen-Scanner ist; ein Nukleinsäure-Hybridanalysator oder ein Ultraschallsensor ist, der dazu ausgelegt ist, das Volumen der Probe zu bestimmen.

12. Analysatorvorrichtung nach einem der vorhergehenden Ansprüche, wobei die rekursiven Strukturelemente ein diagnostisches Testsystem bilden, das Reagenzien, um eine diagnostische Analyse durchzuführen, wie beispielsweise einen Puffer, ein Peptid, ein Protein, ein Enzym, ein Nukleotid, einen Antikörper, einen Farbstoff und eine Messeinheit umfasst, um biochemische Reaktionsergebnisse zu detektieren, vorzugsweise ein Photometer, das dazu ausgelegt ist, Farben und/oder Farbveränderungen zu detektieren.

13. Analysatorvorrichtung nach Anspruch 12, wobei das Testsystem ein CRISPR/Cas-Testsystem ist, das mindestens Folgendes umfasst: (i) ein optionales erstes rekursives Strukturelement oder eine Reaktionszone in einem rekursiven Strukturelement, das eine Reverse-Transkriptase-Aktivität, einen Puffer und Nukleotide umfasst; (ii) ein zweites, vorzugsweise benachbartes, rekursives Strukturelement oder eine Reaktionszone in einem rekursiven Strukturelement, das eine Rekombinase, einsträngige DNA-bindende Proteine (SSBs) und eine Strang-verdrängende Polymerase, einen Puffer für RPA und Nukleotide umfasst; (iii) ein drittes, vorzugsweise benachbartes, rekursives Strukturelement oder eine Reaktionszone in einem rekursiven Strukturelement, das eine Transkriptase, vorzugsweise eine T7-Transkriptase, einen geeigneten Puffer und Nukleotide umfasst; und (iv) ein viertes, vorzugsweise benachbartes, rekursives Strukturelement oder eine Reaktionszone in einem rekursiven Strukturelement, das ein aktiviertes RNA-bindendes Enzym der Cas-Proteinfamilie, vorzugsweise ein Cas13a-Enzym, einen oder mehrere RNA-Reporter, vorzugsweise einen RNA-Reporter, der einen Farbstoff und ein Quencher-Element umfasst, und einen Puffer umfasst; und gegebenenfalls eine Messeinheit, vorzugsweise ein Fluorimeter.

14. Analysatorvorrichtung nach einem der vorhergehenden Ansprüche, wobei eines oder mehrere der rekursiven Strukturelemente eine Mikrosteuerung umfasst.

15. Analysatorvorrichtung nach Anspruch 14, wobei die Mikrosteuerung ein optisches und/oder akustisches Warnsystem steuert, das zum Angeben der Qualität und/oder Richtigkeit eines oder mehrerer der folgenden Parameter programmiert ist: Temperatur, pH-Wert, elektrische Leitfähigkeit, Luftfeuchtigkeit, Position der Analysatorvorrichtung, Operationssequenz, Bewegung der Proben, Bewegungsrichtung der Vorrichtung, Status des Leerens und/oder Füllens eines rekursiven Strukturelements, Blockierung von Verbindungen zwischen rekursiven Strukturelementen oder von externer mechanischer Einwirkung beim Betrieb.

16. Analysatorvorrichtung nach einem der vorhergehenden Ansprüche, wobei sich die Analysatorvorrichtung manuell oder automatisch, vorzugsweise über ein Drehelement, das an mindestens einer Seite der Analysatorvorrichtung verbunden ist, oder durch ein Walzenelement, in einem 3D-Koordinatensystem dreht.

17. Verfahren zum Herstellen einer Analysatorvorrichtung nach einem der vorhergehenden Ansprüche, wobei das Verfahren 3D-Drucken der verschachtelten rekursiven Strukturelemente der Analysatorvorrichtung und der unmittelbaren oder indirekten Verbindungen zwischen den rekursiven Strukturelementen umfasst, wobei die rekursiven Strukturelemente geeignet sind, um Flüssigkeiten darin zu halten, wobei alle rekursiven Strukturelemente Teil eines Fluidverbindungssystems sind, das unmittelbare oder indirekte Verbindungen zwischen den rekursiven Strukturelementen bereitstellt, wobei das Druckverfahren mindestens einmal unterbrochen wird, um eine vorweggenommene Messeinheit und gegebenenfalls ein oder mehrere vorweggenommene Bauteile der Analysatorvorrichtung einzuführen, wie beispielsweise eine Mikrosteuerung, ein Reagenz, ein Peptid, ein Protein, ein Enzym, einen Antikörper, eine Zusammensetzung, eine Energiequelle, eine Heizeinheit, eine Kühleinheit, eine Zeitgebereinheit, eine Sterilisationseinheit und/oder ein Magnetrührwerk;
wobei die Verbindungen die Gestalt gerader oder gekrümmter Verlängerungen mit zwei Öffnungen aufweisen.

18. System, die Analysatorvorrichtung nach einem der Ansprüche 1 bis 16 und mindestens ein Reagenzpaket umfassend, wobei das Reagenzpaket als Patrone bereitgestellt wird, das vorzugsweise einen Puffer, eine Säure, eine Base, ein Peptid, ein Protein, ein Enzym, einen Antikörper und/oder einen Farbstoff umfasst.

19. Verfahren zur In-vitro-Diagnose einer Virus- oder Bakterieninfektion oder von Krebs, die diagnostische Analyse einer Probe in einer Analysatorvorrichtung nach einem der Ansprüche 1 bis 16 oder das System nach Anspruch 18 umfassend.

20. Verfahren nach Anspruch 19, einen oder mehrere der folgenden Schritte umfassend
(i) Reinigen der Probe vor einer Analyse;
(ii) Durchführen einer Reaktion mit der Probe in einem oder mehreren rekursiven Strukturelementen nach einem der Ansprüche 1 bis 17;
(iii) Detektieren von Reaktionsergebnissen, vorzugsweise durch Beobachtung in einem Fenster oder über eine Messeinheit.

21. Verwendung der Analysatorvorrichtung nach einem der Ansprüche 1 bis 16 oder des Systems nach Anspruch 18 zur diagnostischen Analyse einer Probe.

22. Verwendung nach Anspruch 21, wobei die diagnostische Analyse die CRISPR/Cas-basierte Diagnose einer Virus- oder Bakterieninfektion oder von Krebs ist.

## Revendications

1. Dispositif analyseur pour analyser un échantillon comprenant
(i) au moins deux éléments imbriqués de structure récurrente ;
(ii) dans lequel chacun des éléments de structure récurrente a au moins une connexion à au moins l'un des autres éléments de structure récurrente ; et
(iii) au moins une fonctionnalité de mesure ;
dans lequel le dispositif analyseur est configuré pour permettre, par l'intermédiaire des connexions, un déplacement de l'échantillon et de matière présente dans un élément de structure récurrente, telle que de la matière liquide, à l'élément suivant de structure récurrente par gravité, sous la forme d'une rotation du dispositif analyseur dans un système de coordonnées en 3D ou, éventuellement, par des forces supplémentaires telles qu'un mouvement capillaire ou une pression d'air ou de liquide, dans lequel au moins l'un des éléments de structure récurrente comprend au moins une ouverture à la surface, configurée pour recevoir et/ou libérer l'échantillon pour analyse, dans lequel les éléments de structure récurrente conviennent pour renfermer des liquides en leur sein, dans lequel tous les éléments de structure récurrente font partie d'un système de communication fluidique procurant des connexions directes ou indirectes entre des éléments de structure récurrente, dans lequel le dispositif analyseur comprend, au sein d'au moins un élément de structure récurrente ou dans des éléments distincts de structure récurrente, une zone de mesure mettant en œuvre la fonctionnalité de mesure ; et dans lequel les connexions ont la forme de deux parties oblongues droites ou incurvées ayant deux ouvertures.

2. Dispositif analyseur suivant la revendication 1, dans lequel les, de préférence tous les éléments de structure récurrente, communiquent par au moins une goulotte comprenant une ouverture à la surface du dispositif, configurée pour recevoir une cartouche comprenant un ou plusieurs réactifs sous la forme d'une pile de réactif et/ou d'un ou de plusieurs échantillons.

3. Dispositif analyseur suivant la revendication 2, dans lequel l'ouverture peut être scellée, de préférence par une valve, un obturateur, un couvercle, un bouchon ou une bande adhésive.

4. Dispositif analyseur suivant l'une quelconque des revendications précédentes, dans lequel l'élément de structure récurrente comprend, consiste essentiellement ou consiste en une matière, qui peut être imprimée par une imprimante en 3D, de préférence une matière plastique ou métallique.

5. Dispositif analyseur suivant la revendication 4, dans lequel la matière plastique est une matière thermoplastique, une matière plastique thermodurcissable ou une résine activée par la lumière.

6. Dispositif analyseur suivant l'une quelconque des revendications précédentes, dans lequel les connexions directes ou indirectes entre les éléments de structure récurrente peuvent être fermées d'une manière commandée.

7. Dispositif analyseur suivant l'une quelconque des revendications précédentes, dans lequel l'échantillon pour analyse est un échantillon liquide minéral ou organique, de préférence en échantillon de fluide corporel, tel que du sang entier, du sérum sanguin, du plasma sanguin, du crachat ou de l'urine.

8. Dispositif analyseur suivant l'une quelconque des revendications précédentes, dans lequel la forme du dispositif analyseur est un prisme ayant de trois à dix côtés, un cylindre ou un cuboïde.

9. Dispositif analyseur suivant l'une quelconque des revendications précédentes, dans lequel le dispositif analyseur comprend en son sein au moins un élément de structure récurrente ou dans des éléments distincts de structure récurrente, une zone de réaction ou une zone formant réservoir ou leurs combinaisons.

10. Dispositif analyseur suivant l'une quelconque des revendications précédentes, dans lequel le dispositif analyseur comprend en outre au sein d'au moins un élément de structure récurrente ou dans des éléments distincts de structure récurrente ou en connexion avec au moins un élément de structure récurrente : une unité de mesure mettant en œuvre la fonctionnalité de mesure, une source d'énergie telle qu'une batterie ou un accumulateur, une unité de chauffage, une unité de refroidissement, une unité d'horloge, une unité de stérilisation, un agitateur magnétique, un système sous vide, un hublot d'observation, une entrée de gaz et/ou des sorties de gaz.

11. Dispositif analyseur suivant la revendication 10, dans lequel l'unité de mesure est un magnétomètre ou un capteur gyroscopique configuré pour déterminer le changement de position du dispositif analyseur ; un hygromètre configuré pour déterminer l'humidité à l'intérieur ou à l'extérieur du dispositif analyseur ; une unité de mesure de la température configurée pour déterminer la température en diverses positions, dans ou sur le dispositif analyseur, de préférence un capteur infrarouge, un résonnateur à quartz ou une résistance de mesure au platine ou un capteur de température à bande interdite au silicium ; un photomètre configuré pour détecter des couleurs et/ou des changements de couleur ; un pH-mètre ; une unité de mesure de la conductivité électrique ; un spectromètre Raman ou un spectromètre IR configuré pour analyser la composition ou la granulométrie de l'échantillon ; un viscosimètre ; un tomodensitomètre d'imagerie par résonnance magnétique ; un tomodensimètre aux rayons X ; un analyseur hybride d'acide nucléique ou un capteur d'ultrasons configuré pour déterminer le volume de l'échantillon.

12. Dispositif analyseur suivant l'une quelconque des revendications précédentes, dans lequel l'élément de structure récurrente constitue un système de test de diagnostic, qui comprend des réactifs pour effectuer une analyse de diagnostic, tels qu'un tampon, un peptide, une protéine, un enzyme, un nucléotide, un anticorps, un colorant et une unité de mesure pour déterminer des résultats de réactions biochimiques, de préférence un photomètre configuré pour détecter des couleurs ou des changements de couleur.

13. Dispositif analyseur suivant la revendication 12, dans lequel le système de test est un système de test CRISPR/Cas comprenant au moins : (i) un premier élément de structure récurrente facultatif ou une zone de réaction au sein d'un élément de structure récurrente, qui comprend une activité de transcriptase inverse, un tampon et des nucléotides ; (ii) un deuxième élément de structure récurrente, de préférence voisin ou une zone de réaction au sein d'un élément de structure récurrente, qui comprend une recombinase, des protéines de liaison d'ADN à simple brin (SSBs) et une polymérase de déplacement de brin, un tampon pour RPA et des nucléotides ; (iii) un troisième élément de structure récurrente, de préférence voisin, ou une zone de réaction au sein d'un élément de structure récurrente, qui comprend une transcriptase, de préférence une transcriptase T7, un tampon approprié et des nucléotides ; et (iv) un quatrième élément de structure récurrente, de préférence voisin, ou une zone de réaction au sein d'un élément de structure récurrente, qui comprend un enzyme de liaison d'ARN activé de la famille de protéine Cas, de préférence un enzyme Cas13a, un ou plusieurs reporters d'ARN, de préférence un reporteur d'ARN qui comprend un colorant et un élément désactivateur, et un tampon ; et éventuellement une unité de mesure, de préférence un fluoromètre.

14. Dispositif analyseur suivant l'une quelconque des revendications précédentes, dans lequel un ou plusieurs des éléments de structure récurrente comprend une micro-unité de commande.

15. Dispositif analyseur suivant la revendication 14, dans lequel la micro-unité de commande commande un système optique et/ou audible d'avertissement pour indiquer la qualité de l'un ou plusieurs des paramètres suivant et/ou qu'ils sont corrects : température, pH, conductivité électrique, humidité, position du dispositif analyseur, séquence des opérations, déplacement d'échantillons, direction de déplacement du dispositif, statut de vidange et/ou de remplissage d'un élément de structure récurrente, blocage de connexions entre des éléments de structure récurrente ou impact mécanique externe pendant un fonctionnement.

16. Dispositif analyseur suivant l'une quelconque des revendications précédentes, dans lequel le dispositif analyseur tourne manuellement ou automatiquement, de préférence par l'intermédiaire d'un élément tournant relié à au moins un côté du dispositif analyseur, ou par un élément à rouleau, dans un système de coordonnées en 3D.

17. Procédé de production d'un dispositif analyseur tel que défini dans l'une quelconque des revendications précédentes, dans lequel le procédé comprend une impression en 3D des éléments imbriqués de structure récurrente du dispositif analyseur et les connexions directes ou indirectes entre les éléments de structure récurrente, dans lequel les éléments de structure récurrente conviennent pour renfermer des liquides en leur sein, dans lequel tous les éléments de structure récurrente font partie d'un système de communication fluidique procurant des connexions directes ou indirectes entre les éléments de structure récurrente, dans lequel l'opération d'impression est interrompue au moins une fois pour introduire une unité de mesure préfigurée et éventuellement une ou plusieurs parties préfigurées du dispositif analyseur, telles qu'une micro-unité de commande, un réactif, un peptide, une protéine, un enzyme, un anticorps, une composition, une source d'énergie, une unité de chauffage, une unité de refroidissement, une unité d'horloge, une unité de stérilisation et/ou un agitateur magnétique ; dans lequel les connexions ont la forme de parties oblongues rectilignes ou incurvées ayant deux ouvertures.

18. Système comprenant le dispositif analyseur tel que défini dans l'une quelconque des revendications 1 à 16 et au moins une pile de réactif, dans lequel la pile de réactifs est prévue sous la forme d'une cartouche, comprenant de préférence un tampon, un acide, une base, un peptide, une protéine, un enzyme, un anticorps et/ou un colorant.

19. Procédé de diagnostic in vitro d'une infection virale ou bactérienne ou d'un cancer, comprenant l'analyse diagnostic d'un échantillon dans un dispositif d'analyse suivant l'une quelconque des revendications 1 à 16 ou dans le système suivant la revendication 18.

20. Procédé suivant la revendication 19 comprenant un ou plusieurs des stades
(i) purifier l'échantillon avant analyse ;
(ii) effectuer une réaction avec l'échantillon dans un ou plusieurs éléments de structure récurrente, tels que définis à l'une quelconque des revendications 1 à 17 ;
(iii) détecter des résultats de réaction de préférence par observation dans un hublot ou par l'intermédiaire d'une unité de mesure.

21. Utilisation du dispositif d'analyse suivant l'une des quelconque des revendications 1 à 16 ou du système suivant la revendication 18, pour l'analyse de diagnostic d'un échantillon.

22. Utilisation suivant la revendication 21, dans laquelle l'analyse de diagnostic est le diagnostic à base de CRISPR/Cas d'une infection virale ou bactérienne ou du cancer.
